# EUROPEAN PATENT APPLICATION

(11) **EP 4 129 051 A1**
(43) Date of publication of application: **08.02.2023**
(21) Application number: 22182413.9
(22) Date of filing: 04.08.2016
(51) Int. Cl.: A01H 1/04, C12Q 1/68, C12N 15/82

(54) **BRASSICA PLANT WITH POD SHATTERING TOLERANCE**

(30) Priority: 11.08.2015 EP 15306287
(62) Divisional of application: 16750738.3
(71) Applicant: Limagrain Europe, 63360 Saint-Beauzire (FR)
(72) Inventor: ABEL, Stefan, 31228 Peine (DE); HANNETON, Laurent, 77390 Verneuil l'Etang (FR); GEGAS, Vasilis, Rothwell LN7 6 DT (GB); COMADRAN, Jordi, 63200 Riom (FR); MARTINANT, Jean Pierre, 63910 Vertaizon (FR)
(74) Representative: Plasseraud IP

(57) **Abstract**

The field of the invention is related to plant breeding, particularly the development of new Brassica plants with pod shattering tolerance. The invention provides in particular a Brassica plant comprising a Raphanus genomic fragment within its genome, wherein said fragment confers pod shattering tolerance phenotype POSH⁺ and said fragment is characterized by the absence of at least one SNP within one or more of the following Raphanus markers: SEQID NOs 4-18.

## Description

The field of the invention is related to plant breeding, particularly the development of new Brassica plants with pod shattering tolerance.

### BACKGROUND OF THE INVENTION

Rape culture, also called canola across the Atlantic, is widespread on all continents due to the fact of its multiple strengths in both the food and industrial sectors. Indeed, rapeseed produces a large oil widely used as a food product but also as a biofuel especially in the automotive industry or the like. Rape also allows the production of cakes that are a good source of protein in animal feed (cattle, pigs and poultry).

Despite these strengths, the food use of oilseed rape oil has long been restricted because of his excessive erucic acid content. Indeed, based on rapeseed varieties, the level of erucic acid could be up to 50% of total fatty acids of the plant and have a detrimental impact on human health.

Similarly, the use of rapeseed for the manufacture of meals has also long been hampered due to the high content of glucosinolates in the seed. When forming cake, grinding seeds frees the myrosinase enzyme that converts glucosinolates seeds in various by-products such as glucose, thiocyanates, isothiocyanates and nitriles that may lead to metabolic disorders in mammals. The extensive development of rape crop is mainly due to two major technical advances: the decrease of the level of erucic acid in the oil and the reduction of the level of glucosinolates in the seed. Indeed, today, a network of plant breeding has produced commercial varieties whose level of erucic acid is less than 2% of total fatty acids of the rapeseed plant. Moreover, in Europe, the Decree 2294/92 has set the maximum acceptable rate of glucosinolates in seed 25 micromol per gram of seed at 9% humidity. Glucosinolates rate and the level of erucic acid being two interesting parameters in the production of products derived from rapeseed, breeders have therefore sought to develop varieties called "double zero" that is to say the varieties rapeseed which present very low level of erucic acid in the oil and low levels of glucosinolates in the seeds.

Although the oilseed traits are of major importance for Brassica breeding activities, other agronomic traits are also selected in order to improve the creation of competitive new varieties. For example, disease resistance, yield, morphological trait like silique length or physiological trait like male sterility, fertility restoration or shattering tolerance.

Pod shattering is agronomically important because it may result in the premature shedding of seed before the crop can be harvested. Adverse weather conditions can exacerbate the process resulting in a greater than 50% loss of seed. This loss of seed not only has a dramatic effect on yield but also results in the emergence of the crop as a weed in the subsequent growing season.

Arabidopsis mutants have been used to better understand the genetic determinism of pod shattering. Different genes encoding for transcription factors have been identified to be involved in the regulation of pod shattering tolerance. For example, SHATERPROOF 1 (SHP1) and 2 (SPH2), NAC, INDEHISCENT (IND), ALCATRAZ (ALC) are involved in valve-margin development. REPLUMLESS (RPL) and FRUITFULL(FUL) are involved in repressing the expression of valve-margin identity genes. FUL and IND function have been validated through ectopic expression.

Natural genetic variation for shatter tolerance has been identified in oilseed crop species like Brassica napus. One major locus was identified from a F2 population derived from Chinese parental lines of B. napus and has been mapped on chromosome A09 (Hu Z et al, 2012 - Discovery of pod shatter-resistant associated SNPs by deep sequencing of a representative library followed by bulk segregant analysis in rapeseed - PLoS ONE 7:e34253). Recently, experiments carried on a biparental population and a diverse germplasm panel from Brassica napus allowed the identification of few QTLs for pod shattering tolerance. They have been mapped on different chromosomes of Brassica napus genome (Raman et al, 2014, Genome-wide delineation of natural variation for pod shatter resistance in Brassica napus - PLoS ONE 9, 7, e101673). Interestingly, the authors conclude on the difficulties to demonstrate the function of the allelic variation in conferring pod shattering resistance notably due to the expected high level of number of copy of the different genes involved in pod shattering and the complexity of their organization in the genome.

Rape is a self-pollinating species, these varieties have long been only population varieties, not hybrids. However, the development of hybrid plants has multiple interests rape for both the farmer and the breeder, since it allows to obtain improved plants, exhibiting qualities of heterosis (or hybrid vigor), homeostasis (stability of the plant in different environments), the possibility of introducing and combining resistance genes to insects, fungi, bacteria or viruses, or adaptation to abiotic stress. But this development of rapeseed hybrids requires effective means of pollination control. To do this, cytoplasmic male sterility (or CMS Cytoplasmic Male Sterility) systems have been developed such as Polima, and especially Kosena and Ogura systems.

The Ogura cytoplasmic male sterility system is based on the use of a determinant of male sterility derived from the cytoplasm of radish (Raphanus sativus), which was transferred from the radishes in Brassica napus by inter-specific crosses, bailouts of embryos and backcrosses (Bannerot et al, 1974). Protoplast fusion was needed to produce cytoplasmic male sterile hybrids (Pelletier et al., 1983). But CMS Ogura cytoplasmic male sterility is dominant, hybrid rapeseed plant does not produce pollen, and without pollen, the plant does not produce seeds. To remedy this situation and get a harvest, it is necessary that the male parent of the hybrid contains a gene restoring the male fertility. Such a male fertility restorer gene of Ogura system was identified in radish Raphanus sativus and Brassica plants transferred to the carrier of cytoplasmic male sterility by the National Institute of Agronomic Research in 1987 (Pelletier et al, 1987, Proc 7th Int. Rapeseed Conf. Poznan, Poland, 113/119). Rf restorer gene has been described in the WO92 / 05251 patent application and in Delourme et al, 1991, Proc 8th Int. Rapeseed Conf. Saskatoon, Canada, 1506/1510. However, the resulting plants carrying this Rf gene restoring the male fertility have two major disadvantages: a significant increase in glucosinolates in the seed and a significant decrease of the agronomic characteristics of the plant such as a decrease in the amount of seeds produced, decreased disease resistance and increased susceptibility to lodging. These disadvantages appear to be directly linked to the wearer introgression fragment including the gene Rf restoration of the cytoplasmic male fertility transferred from Raphanus sativus. This chromosomal region not having the Rf-restoring gene, it also comprises one or more genes that result in the abovementioned disadvantages. To remedy this situation, various research programs have sought recombination events in this chromosomal region, recombination to break the existing linkage between the DNA segments encoding the various characters. Although research has been hampered by the fact that the chromosomal region surrounding the restorer gene Rf is very difficult to subject to recombination, different patent applications describe the generation of recombination event in the Raphanus fragment leading to new recombinant lines harboring the Rf gene and a reduced level of glucosinolates and better pod size (see WO97 / 02737, WO98 / 27806, WO 2005/002324, WO 2005/074671, and WO2011020698). Each document describes the generation of specific recombination event between the Rf gene restoring the male fertility and the genes linked to high levels of glucosinolates in the seeds or genes linked to small pod size and each event is characterized by using specific markers.

Low glucosinolate levels in seeds and a good pod size are necessities for any commercialized plant. One effective way to reduce the glucosinolate levels and to improve the pod size in Ogura restorers is to shorten the Raphanus introgression. On the other hand the reduction of the size of the Raphanus fragment may lead to the elimination of agronomic traits of interest. One of these agronomic traits lost after Raphanus fragment size reduction is pod shattering tolerance which is of big importance to reduce the seed losses before harvest and a main advantage of Ogura hybrids. New development in this region is therefore needed but are strongly hampered by the very low recombination rate in the Raphanus fragment, by the lack of any Raphanus genome mapping or sequence and therefore by the lack of any marker specific to this fragment and finally by the complexity of the Brassica genome.

In this context, one of the essential objectives of the invention is to obtain a Brassica plant overcoming all the disadvantages mentioned above. In particular, one objective is to obtain a Brassica plant comprising a shortened Raphanus fragment including the pod shattering tolerance alleles. Said Brassica plant may advantageously be used for breeding to readily transfer such pod shattering tolerance alleles to other Brassica plant with other genetic background. In particular, one objective of the present invention is to provide a Brassica plant comprising a shortened Raphanus fragment including the pod shattering tolerance alleles and the male fertility restoration Rf0 gene.

Another objective of the invention is to identify a new Raphanus pod shattering tolerance that can be used in Brassica breeding activities.

Yet another objective of the invention is to obtain a Brassica plant comprising the Raphanus pod shattering tolerance and the use of this plant to drive the introgression pod shattering tolerance in Brassica plants harboring a pod shattering tolerance phenotype.

Another object of the invention is to obtain seeds, hybrid plants and progeny of said Brassica plants.

The invention also relates to methods for identifying the presence of said pod shattering tolerance allele in Brassica plants and in particular, suitable markers associated (or not associated) to said new pod shattering tolerance.

### SUMMARY

It is therefore disclosed herein a Brassica plant comprising a Raphanus genomic fragment within its genome, wherein said fragment confers pod shattering tolerance phenotype POSH⁺ and said fragment is characterized by the absence of at least one Raphanus SNP within the at least one of the following markers: SEQ ID NOs 4-18.

In specific embodiment of said Brassica plant, the Raphanus genomic fragment is further defined by the presence of at least one of the Raphanus SNP within SEQIDN°19, SEQIDN°20 or SEQIDN°21 marker. For example, said Raphanus SNPs within SEQ ID NO:9 and SEQ ID NOs:12-18 markers are absent. For example, said Raphanus SNPs within all markers of SEQ ID NOs:4-18 are absent.

In another specific embodiment, the Brassica plant as disclosed herein further comprises a Raphanus FRUITFULL allele. Typically, said Raphanus FRUITFULL allele comprises the Raphanus SNP within marker SEQIDN°22.

In specific embodiments, said Brassica plant as disclosed above further comprises the male fertility restoration locus Rf0 within the Raphanus fragment.

In other specific embodiments, the Brassica plant as defined above comprises a CMS Ogura cytoplasm.

It is further disclosed herein a hybrid Brassica plant obtained by crossing a Brassica plant having a Raphanus fragment conferring POSH+ phenotype as disclosed above, with another Brassica plant which does not have said Raphanus fragment conferring POSH+ phenotype, wherein said hybrid plant comprises the Raphanus genomic fragment which confers pod shattering tolerance phenotype POSH⁺.

The seed, or part of plants or their progenies of said Brassica plant are also disclosed herein.

Another aspect disclosed herein relates to methods for identifying a POSH+ Brassica plant as described above, wherein said Brassica plant is identified by detecting the presence of one or more of the Raphanus SNPs within at least one of the following markers SEQIDN°19, SEQIDN°20, SEQIDN°21, and/or by the absence of the Raphanus SNPs within at least one of the following markers: SEQ ID NOs 4-18.

In specific embodiments of such method, the POSH+ Brassica is identified by using a Raphanus fruitfull allele, and more specifically the Raphanus marker SEQIDN°22.

Another aspect disclosed herein relates to new means for detecting one or more Raphanus SNP within one or more of the following markers: SEQ ID NOs 4-22.

Such means may typically be nucleic acid probes or primers or a set of primers or their combinations, for example one or more primers including any of SEQ ID NOs 64-99, 106-108, 112-114 and 52-54.

Such Brassica plants or seeds as disclosed herein are useful for food applications, preferably for oil production and for feed applications, preferably for cake production, or breeding applications for example for use as a parent plant in breeding for improving agronomical value of a Brassica plant, line, hybrid or variety.

It is also disclosed a method of production of a POSH+ Brassica plant, wherein the method comprises the following steps:
a. crossing a first Brassica plant of the present invention as disclosed above with the POSH+ phenotype, with a second POSH⁻ or POSH+ Brassica plant; thereby obtaining a F1 hybrid plant;
b. selfing or backcrossing the F1 hybrid plant with said second POSH⁻ or POSH⁺ Brassica plant;
c. selecting the POSH⁺ Brassica plant among the plant obtained in step b), optionally using at least one Raphanus SNP within at least one of the markers SEQIDN°19, SEQIDN°20 or SEQIDN°21 and
d. optionally, further selecting said POSH+ Brassica plant for the absence of at least one of the Raphanus SNPs within at least one of the markers SEQ ID NOs 4-18.

In specific embodiments of such method, the second POSH⁻ Brassica plant is characterized by the absence of any Raphanus genome fragment within its genome.

In another specific embodiment of such method, the first plant is a plant obtained from a representative sample of the seeds as deposited at NCIMB collection under the number 42444.

It is also disclosed a method of production of a POSH+ Brassica plant of the present invention as described above, wherein the method comprises the following steps:
a. providing a first POSH+ Brassica plant, comprising a Raphanus introgression conferring the POSH+ trait, said Raphanus introgression including at least one of the Raphanus SNP within one or more of the following markers: SEQ ID NO:4-18;
b. crossing said first POSH+ Brassica plant with a second POSH- or POSH+ Brassica plant, thereby obtaining a F1 hybrid plant;
c. selfing or backcrossing the F1 hybrid plant with said second plant POSH⁻ or POSH+;
d. selecting the POSH⁺ plant among the plant obtained in step c), optionally selecting for the presence of at least one Raphanus SNP within at least one of the markers SEQIDN°19, SEQIDN°20 or SEQIDN°21 and/or optionally further selecting for the absence of at least one Raphanus SNP within at least one of the markers SEQIDNOs 4-18.

In specific embodiments of the above method, said first POSH+ Brassica plant comprises the Rf0 Ogura fertility restoration gene.

The disclosure further pertains to the Brassica plant obtainable or obtained by the above methods of production.

### BRIEF DESCRIPTION OF THE FIGURES

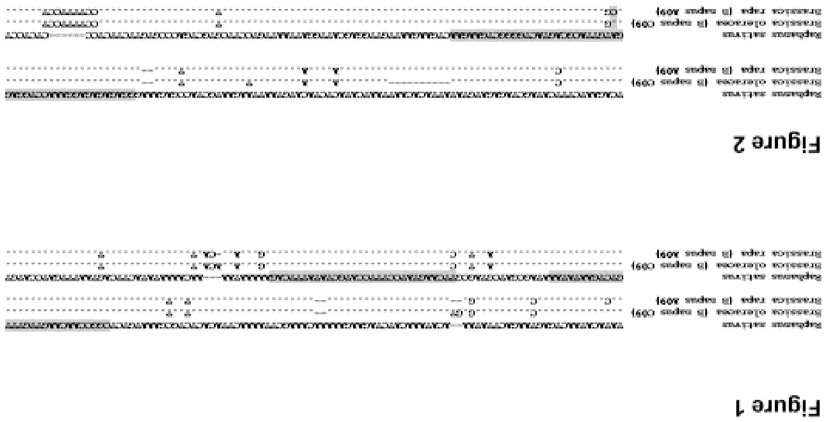

Figure 1 shows the non-genome specific design strategy used for markers FRUITFULL H1 04.
Figure 2 shows the genome specific design strategy used for markers FRUITFULL_spe_01 on the alignment of Raphanus and Brassica sequences.

### DETAILED DESCRIPTION

### The Brassica plant

As used herein, the term "Brassica plant" includes a plant of Brassica species, including B. napus, B. juncea and B. rapa; preferably B. napus.

As used herein, "pod shattering" also referred as "fruit or pod dehiscence" refers to a process that takes place in a fruit after seed maturation, whereby the valves detach from the central septum freeing the seeds. The region that breaks (i.e. the "dehiscence zone") runs the entire length of the fruit between the valves and the replum (external septum). At maturity, the "dehiscence zone" is essentially a non-lignified layer of cells between a region of lignified cells in the valve and the replum. Shattering occurs due to the combination of cell wall loosening in the dehiscence zone and the tensions established by the differential mechanical properties of the drying cells in the silique.

The pod shattering trait is usually measured through laboratory tests that simulate the forces acting on the pods in the natural conditions. Different methods are available as described in Kadkol et al 1984 - Evaluation of brassica accessions for resistance to shatter - Euphytica, 33, 61-71 , Liu et al, 1994 - Pendulum test for evaluation of rupture strength of seed pods- Journal of texture studies, 25, 179-189 or the method as described in example 3 of the present disclosure. Using this last method, the pods are harvested at complete maturity stage (BBCH97). The pod shattering tolerance is corresponding to the tension necessary to tear the two halves of the pod apart.

As used herein, the Brassica plant harboring pod with tension values more or equal to 2.3 Newton (N) are defined as pod shattering tolerant. In the present disclosure, they are also defined as POSH+ referring to the presence of the Raphanus POSH region in the plant genome. In specific embodiments, the POSH+ Brassica plant will harbor pods with a tolerance comprised between 2.3 and 7N (unit). Preferably, the POSH+ Brassica plant will harbor pods with a tolerance comprised between 2.3 and 5 N. More generally a POSH+ Brassica plant can be a Brassica plant harboring within its genome a long introgression of the Raphanus genome or it also can be one of the Brassica plant of the present disclosure.

As used herein, the Brassica plants with pod having tension values less than 2.3 N are defined as not pod shattering tolerant. Particularly, the pod tension will be above 0.6 N. In the present disclosure, they are also referred as POSH-, referring to the absence of the Raphanus POSH+ region in the plant genome. More generally, a POSH- Brassica plant could be fertile or not and for example could comprise or not the Rf0 fertility restorer gene, it could also be sterile or not and for example could comprise or not the Ogura male sterile cytoplasm, and it could also be or not a maintainer plant. Moreover, said POSH- Brassica plant may comprise a Raphanus introgression or no Raphanus introgression.

As used herein, the term "allele(s)" means any of one or more alternative forms of a gene at a particular locus. In a diploid (or amphidiploidic cell of an organism), alleles of a given gene are located at a specific location or locus on a chromosome. One allele is present on each chromosome of the pair of homologous chromosomes.

Whenever reference to a "plant" or "plants" is made, it is understood that also plant parts (cells, tissues or organs, seed pods, seeds, severed parts such as roots, leaves, flowers, pollent, etc.), progeny of the plants which retain the distinguishing characteristics of the parents (especially, pod shattering tolerance associated to the Raphanus fragment), such as seed obtained by selfing or crossing, e.g. hybrid seeds (obtained by crossing two inbred parent plants), hybrid plants and plant parts derived there from are encompassed herein, unless otherwise indicated.

As used herein, a "Raphanus genomic fragment" refers to an introgression, and preferably the original introgression and any of their recombinant fragment of the Raphanus sativa genome within the Brassica napus genome, which introgression is found in many commercialized Brassica varieties, including without limitation Albatros or Artoga varieties. For ease of reading, the original introgression will be defined hereafter as the "long introgression of the Raphanus genome".

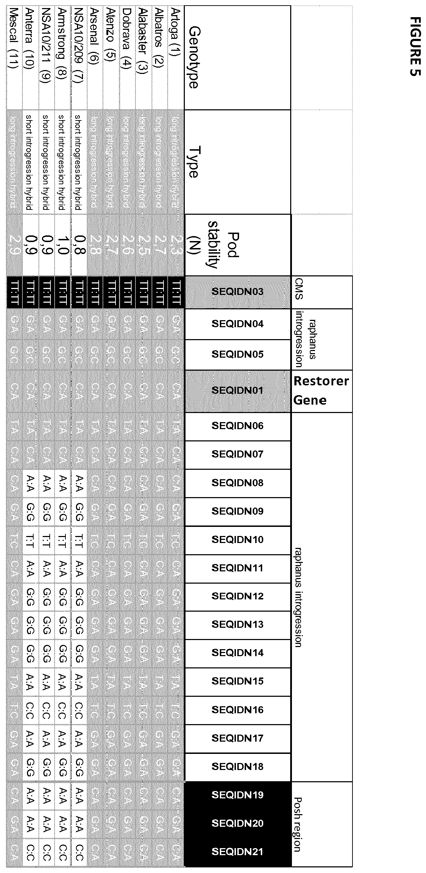

Such long introgression of the Raphanus genome within the Brassica napus genome further comprises the Rf0 gene for the fertility restoration of the Ogura CMS system. This Raphanus long introgression may not comprise any Brassica napus genome fragments. Examples of commercialized Brassica varieties comprising a long Raphanus introgression fragment are depicted in figure 5 like Albatros or Artoga.

A shorter introgression of the Raphanus genome within the Brassica napus genome have also been described in the art and said introgression comprises the Rf0 gene for the fertility restoration of the Ogura CMS system but the inventors now identified that such shorter introgression did not comprise a genome region conferring the pod shattering tolerance named POSH+. Examples of commercialized Brassica varieties comprising such shorter Raphanus introgression fragment is listed in figure 5 like Anterra or are also described in patent application WO2011/020698, WO97/02737, WO98/27806, WO 2005/002324 or WO 2005/074671.

As used herein, the term "introgression" refers to a DNA fragment of a particular species, in the present case, from Raphanus sativus species, and transferred into another plant species, in the present case, Brassica, more preferably Brassica napus.

As used herein a "marker" refers to a specific DNA sequence identified within the genome of a plant and which can be used to determine whether a plant has inherited a particular phenotype or allele of interest from a parent plant. Said marker may include coding or non-coding sequences. In particular, said marker may include one or more Single Nucleotide Polymorphism or SNP identified between the Raphanus and the napus genome. It is also possible to identify sequence deletion/insertion (indel) polymorphism. In the present invention, the rapa genome is not considered, therefore the napus genome will also be identified as oleracea genome.

As used herein, a "Raphanus SNP" corresponds to the nucleotide present in the Raphanus genome at a polymorphic position compared to the oleracea genome.

It is herein disclosed Raphanus SNPs within markers (identified by their nucleotide sequence) for determining, in a Brassica plant, whether any recombinant fragment of the long Raphanus introgression further retains the POSH+ allele conferring pod shattering tolerance. Accordingly, the Brassica plant of the present disclosure includes a recombinant fragment of said long introgression, which is advantageously shorter than the long introgression while retaining at least the POSH+ allele.

More specifically, certain Raphanus SNPs found in said Raphanus long introgression have been characterized as not being linked to the POSH+ allele. Such SNPs are included in any of the following fifteen markers: SEQ ID NOs 4-18.

Accordingly, a Brassica plant according to the present disclosure comprises a Raphanus genomic fragment within its genome, wherein said fragment confers pod shattering tolerance phenotype (POSH⁺) and said fragment is characterized by the absence of at least one Raphanus SNP within at least one of the following markers: SEQ ID NOs 4-18.

For each of these markers, a Raphanus SNP has been identified (see Table 2). Preferably 1, 2, 3, 4, 5, 6, 7, 8, 9, 10, 11, 12, 13, 14, 15 or all of these SNPs are absent in said Brassica plant.

Other markers, and in particular Raphanus SNPs have been found in said Raphanus long introgression and characterized as being linked to the POSH+ allele. Such Raphanus SNPs are identified in any of the following three sequence markers: SEQIDN°19, SEQIDN°20 or SEQIDN°21.

In a specific embodiment, said Brassica plant comprises a Raphanus genome fragment within its genome, but not the region including the Raphanus SNPs within the markers: SEQ ID NOs 12-18 and SEQIDN°9.

In a specific embodiment, said Brassica plant comprises a Raphanus genome fragment within its genome, but not the region including the Raphanus SNPs within the markers: SEQ ID NOs 4-18.

In another specific embodiment that may be combined with the previous embodiments, said Brassica plant comprises at least the region of the Raphanus genome fragment including 1, 2 or 3 of the Raphanus SNPs within the following markers: SEQIDN°19, SEQIDN°20 or SEQIDN°21, said SNP being identified in Table 1.

Alternatively, in another specific embodiment, said Brassica plant comprises at least the region of the Raphanus genome fragment including the Raphanus FRUITFULL allele, as identified within the following marker: SEQIDN°22 or SEQ ID NO:31.

In another specific embodiment, the Brassica plant is non-transgenic plant. Transgenic or "genetically modified organisms" (GMO) as used herein are organisms whose genetic material has been altered using techniques generally known as "recombinant DNA technology". Recombinant DNA technology is the ability to combine DNA molecules from different sources into one molecule ex vivo (e.g. in a test tube). This terminology generally does not cover organisms whose genetic composition has been altered by conventional cross-breeding, or by "mutagenesis" breeding. "Non-transgenic" thus refers to plants and food products derived from plants that are not "transgenic" or "genetically modified organisms".

The invention also relates to hybrid Brassica plants which can be produced by crossing a Brassica plant obtained above with a second plant. For example, a hybrid Brassica plant may be obtained by crossing a Brassica plant as disclosed herein which have a Raphanus fragment conferring POSH+ phenotype and another Brassica plant which does not have said Raphanus fragment conferring POSH+ phenotype, wherein said hybrid plant comprises the Raphanus genomic fragment which confers pod shattering tolerance phenotype POSH⁺.

Methods to produce hybrid plants are well-known in the art. Typically, hybrid plants are produced by preventing self-pollination of female parent plants, permitting pollen from male parent plant to fertilize such female parent plant and allowing F1 hybrid seeds to form on the female plants. Self-pollination can be prevented by emasculating the flowers at an early stage of flower development. Alternatively, pollen formation can be prevented on the female parent plants using a form of male sterility. Hybrid plants can be obtained by different genetic systems well known from the person skilled in the art like for example, the CMS systems like Ogura system or the Kosena system (See Yamagashi and Bhat, 2014, Breeding Science, 64: 38-47), or the MSL (Male Sterility Lembke) system (Pinochet et al., 2000 OCL-Leagineux Corps Gras Lipides 7:11-16). Preferably, the hybrid plants of the invention are obtained with the Ogura system.

Therefore, it is also disclosed herein the Brassica plants or lines according to the present disclosure developed to obtain such hybrid plants. Such plants or lines typically comprise the genetic and/or cytoplasmic elements necessary for the implementation of the corresponding hybrid system. Preferably, the plants or lines comprise the fertility restoration gene Rf0 and /or the cytoplasm of the Ogura system.

### Method of producing a Brassica plant with pod shattering tolerance phenotype (POSH+)

The present disclosure also relates to new methods to produce Brassica plants with pod shattering tolerance phenotype (POSH+) as described in the previous section.

In one embodiment, said method comprises the following steps:
a. providing a first POSH+ Brassica plant comprising a Raphanus introgression conferring the pod shattering tolerance POSH⁺; said Raphanus introgression including at least one of the Raphanus SNP within one or more of the following markers: SEQ ID NOs 4-18;
b. crossing said first POSH+ Brassica plant with said second POSH⁻ or POSH+ Brassica plant, thereby obtaining a F1 hybrid plant;
c. selfing or backcrossing the F1 hybrid plant with the second plant POSH⁻ or POSH+;
d. selecting the POSH⁺ plant among the plant obtained in step c), optionally by selecting for the presence of at least one Raphanus SNP within at least one of the markers SEQIDN°19, SEQIDN°20 or SEQIDN°21 and/or optionally further selecting for the absence of at least one Raphanus SNP within at least one of the markers SEQ ID NOs 4-18.

Advantageously, one of the known varieties comprising the long or short introgression can be used as the first Brassica plant. In specific embodiment, the second (recurrent) Brassica that is used in the above method is a Brassica plant characterized by the absence of any Raphanus genome fragment within its genome, i.e; a plant wherein the Raphanus genome fragment have not been introgressed. Alternatively, said second Brassica plant does not contain at least the Rf gene for fertility restoration of the Ogura CMS system and does not contain the pod shattering tolerant region (POSH⁻). This second Brassica plant can be for example any wild type non restorer and POSH⁻Brassica napus plant. Alternatively, said second plant comprises the short introgression, including the Rf0 Ogura fertility restoration gene. In another specific embodiment, the first Brassica plant further includes the Rf0 Ogura fertility restoration gene.

At step c, backcrossing of F1 hybrid plant with the recurrent second plant aims at reducing the percentage of the genome of the recurrent plant and decrease the percentage of genome of the parent plant (containing the Raphanus introgression). Thanks to the selection markers as disclosed in the above methods, it is possible to select/retain the POSH+ phenotype during the selection process.

The applicant has deposited a sample of seeds of the disclosed Brassica plant with said Raphanus introgression conferring the POSH+ trait, under the Budapest treaty, at NCIMB collection under the number 42444.

The present disclosure further includes and provides for methods of identifying a POSH+ Brassica plant as disclosed in the previous section, and more generally methods of selecting or breeding Brassica plants for the presence or absence of POSH+ allele as comprised in the Raphanus introgression, for example, as molecular guided programs. Such methods of identifying, selecting or breeding Brassica plants comprise obtaining one or more Brassica plants and assessing their DNA to determine the presence or absence of the POSH+ allele contained in the Raphanus introgression and/or the presence or absence of other alleles or markers, for example other markers of the Raphanus introgression not associated with the POSH+ allele. Such methods may be used, for example, to determine which progeny resulting from a cross have the POSH+ allele and accordingly to guide the preparation of plants having POSH+ allele in combination with the presence or absence of other desirable traits.

In specific embodiments, determining the presence of the POSH+ allele or other markers, comprises determining the presence of markers of the Raphanus introgression associated to the POSH+ allele and/or the absence of markers of the Raphanus introgression not associated to the POSH+ allele. Accordingly, plants can be identified or selected by assessing them for the presence of one or more individual SNPs appearing in Table 1 for POSH+, and/or the absence of one or more individual SNPs appearing in Table 2 for Raphanus fragment not related to POSH+ allele.

In a specific embodiment, Rf0 locus may further be identified.

**Table 1 : Raphanus SNP associated to POSH+ allele (one identified SNP is highlighted in bold font)**

| SEQ ID | Sequence |
|---|---|
| N°19 | TAGAGCTGAAGCTAGGTATAGGAGGCACATCATAYAAAGATTTCATTCAAAGCCTTCATCTA CCTATGCAATTGAGTCAAGTAGACCCAATAGTAGCGTCCTTCTCYGGAGGAGCTGTTGGTG TGATCTCRGCKYTGATGGTWGTWGAAGTCAACAACGTGAAGCAGCAAGAGCACAAGAGAT GCAAATACTGTCTAGGAA |
| N°20 | TTAAGAACTGTGTCACTGACATTGACCCTGAGAGGGAGAAGGAGAAGAGAGAAAGGATGGA AAGCCAAAACCTCAAGGCTAGTACAAAGCTGAGTCAAGCGAGGGAGAAAATCAAGCGCAAG TATCCACTTCCTGTTGCAAGGAGRCAACTYTCCACTGGRTACNTGGAAGATGCTCTCGAAG AGGATGAAGAGACAGACC |
| N°21 | GCTCAGGTAGATCTCCCACGGGTTGGGGAAGAGGATCCGGATATGGGTATGGGTCTGGAT CTGGATCAGGTAGCGGATATGGGTACGGTTCCGGAGGTGGAGGAGSACGTGGTGGTGGG TATGGTTATGGAAGCGGAAATGGTCGGTCTGGAGGWGGTGGTGGCTCTAATGGTGAA GTTGCCGCTTTGGGCCACGGTG |
| N°22 | GGGAGAGAGAGGAAACCTGGAGGATGTTACGCAGTACTGGGGCTGAAGAACTGAAGAATT GTTGGAGCATTGGATTAATTGTCCTTCKTGCTGACCCGTGTTCTTCT |

**Table 2 : Raphanus SNP associated to Raphanus fragment but not POSH+ allele**

| SEQ ID | Sequence |
|---|---|
| N°18 | AGAAGATGGAGTTCTTGATGTTTGATCTYGATCGGGTTTTGAARCCCGGTGGGTTGTTC TGGTTGGATAACTTCTACTGCGCTAGTGACGTGAAGAAGAAAGAGCTGACGCGTTTGAT YGAGAGGTTTGGGTATAAGAAGCTGAAATGGGTTATTGGAGAGAAGGCTGATGGGCAA GTGWATCTCTCTGCTGTTCTKCAAA |
| N°17 | CACAACATGCCGGTGATTGGTATCCAGCTGACCTTGGATCCAACGATTTCAAAGGTCTC TATGGATATAAGGTCTTTATTGCCATTGCCATTATCCTTGGGGACGGTCTCTACAATCTT GTCAAGATCATTGCTGTCACTGTGAAGGAATTATGCAGCAATAGCTCTAGACACCTCAAT CTACCCGTTGTTRCCAACGTTG |
| N°16 | ACTTTGTTGAYAGYCTTACMGGAGTAGGACTTGTTGATCAAATGGGAAACTTCTTCTGCA AAACGCTCTTGTTTGTGGCTGTAGCTGGAGTTCTTTCATTCGCAAGAACGAAGATTTAG ATAAGCTCAAGGGTCTRWTYGAAGAGACGACGYTRTATGACAAGCARTGGCAAGCGGC TTGGAAAGAGCCGGAAATAATCA |
| N°15 | GTCCATGTTTGATGCAATTGTATCAGCAGACGCATTTGAGAACTTGAAACCAGCTCCAGA TATTTTCTTGGCTGCTTCCAAKATCTTGGGTGTGCCCACATGCGAGTGTATTGTTATTGA AGATGCACTTGCTGGAGTCCAGGCTGCTCAAGCTGCAAACATGAGATGCATAGCTGTGA AAACTACTTTATCTGAAGCAAT |
| N°14 | CTTTTGCTGGTTTTGGTGAAATAGTATCTGTCAAGATACCAGTTGGGAAAGGATGTGGAT TCATTCAGTTTGTCAACAGAGAAAACGCAGAGGAGGCTTTAGAGAAACTAAATGGTTCT GTAATTGGAAAACAAACCGTTCGCCTTTCMTGGGGTCGTAAYCAAGGCAAYAAACAGCC TCGAGGTGGGTATGG |
| N°13 | CTAAGGCAATGAAGTACCTGTCAATAGGTGAAGAAGACGATATATCATGGTCACTTATCA AAGCTGCCTTCTCTTCAGTAGCTCAAACCGCAATCATACCAATGCAAGACATTCTCGGW CTYGGAAGTTCTGCCAGGATGAACACTCCAGCCACTGAGGTGGGAACTGGGGTTGGA GGATTCCGAGTTCAACGAACTTTG |
| N°12 | TTGGCCCTGAAGGTTCTACAGTGCTTCATTATAGACAATCTTCAACTTCTGCTTCTATTG GGAAAATCAGTTGCAAGGTGTACTATTGCAAAGAAGACGAAGTTTGCTTGTACCAGTCT GTTCAGTTTGAGGTACCTTTCAAGRTGGAATCAGAAKCRTCTYCTTCYCAGGTGATCGCA TTCACCGTTAAACCTAGAGCAT |
| N°11 | TCAAGGACTTTGGTGATAGTATTCCAGGACATGGTGGAATCACTGATAGAATGGACTGC CAGATGGTAATGGCAGTATTTGCTTACATATATCTCCAGTCCTTTATCGTCTCCCAAAGC GTTTCGGTTGACAAAATCCTGGACCAGATATTGACGAACCTTAGCTTCGAGGAACAACA AGCTCTCTTCACTAGATTAGGGC |
| N°10 | CTCCTCCKCCGAATCCGTTTGGGGAYGCGTTCAAGGGGCCMGAGATGTGGGCSAAGCT GACGGCGGATCCGTCGACGAGGGGGTTCTTGAAGCAGCCTGACTTCGTCAACATGATG CAGGAGATCCAGAGGAACCCCTAGCAGTCTCAATCTCTACTTGAAGGACCAGAGGGTGAT GCAGTCTCTYGGGGTTTTGTTGAATG |
| N°9 | AGTATGAAGAAGAGGGYGAGTATGAGAGAGGTGGGTCGAAGCAGGAGAGGAGAGT CAGAGGAAGGKCATGGRTACTACGAAGGGCGTAGTAGACGTTCAAGCCATTATGAGCG TGAGGAGGAACAAGGAGGTGASCAAGACCGKTACGAYGACCGTTATGGGAGAGTGGAG GAAGAAGAATACCGTTATGATGATCGTG |
| N°8 | TCAAGAAGACTTACCCAACAGTCCAGCTTACAGCATGGACATTTTTCCCCATTGTGGGAT GGGTAAAYTACAAGTATGTGCCACTGCACTTCCGGGTCATCTTGCACAGCCTCGTYGCA TTCTTCTGGGGAATCTTCCTGACCCTGCGAGCAAGGTCAATGACACTAGCTTTGGCAAA GGCTAAGTGATCAGGGAAACACA |
| N°7 | CTAGTTTCAGGGAATGGTTTRCAGAAGGTTGAATTGATGAAGACGAGAGCTTCTTCATCA GACGAGACCTCAACGTCCATTGACACCAACGAACTCTTTACWGACTTGAAGGAAAAGTG GGATGGTCTTGAGAACAARACRACYGTGGTTATCTAYGGAGGAGGAGCCATTGTWGCT GTTTGGTTATCTTCCATTCTTGTT |
| N°6 | GAAGTGTTCTGGACACAGCTGAGAAAGCCCACGAAGGGGATATCACATGCATTTCGTG GGCACCCAAGGCAATGACAGTTGGGGAGAGAAAGGCGCAGGTATTAGCGACAGCAGG GGTTGACAARAAAGTGAAGCTGTGGGAAGCTCCAAMGTTGCAGTCTGTGTAGACTTGCT ACTGCTGCTGCAATACAAAGAAAGTCT |
| N°5 | TAAAGTATACTCGAAATGGCCCAAATCTCACTCTTTCAAGATCGGCGACTCCCTCTTGTT CTTGTACCCACCAAGCGAAGATTCAATGATTCAAGTGACACCTTCCAACTTCAAGAGCTG CAACACCAAAGATCCGATCTTGTACATGAACGACGGCAACTCTCTCTTCAACCTCACCC AAAACGGAACCTTTTACTTCAC |
| N°4 | TCAGACTCATCCAGATAAAGAAGAACAAAATCTCATCTTCTGTGCACTCTATGGTACAAA CTCCTTCAGGTACAGCWCGAACGCACAGGTTTGCCACTGAAACAGCCGAGCTCCCTGC GCAAGAAGGAGAAAGAGTGACAATTGCATCTGCTGCTCCATCAGATGTTTACAGACAAG TGGGACCTTTCAAGTTTACCCCCA |

A specific Raphanus SNP within each of the above marker sequences in Table 1 and Table 2 have been shown under bold font. Of course, the skilled person may use other Raphanus SNPs identified within the above markers as depicted in Table 1 and Table 2. Some of these SNPs are indicated by the IUPAC code in the above sequence.

More generally, it is disclosed herein the specific means for detecting the POSH+ allele of the Raphanus introgression in a plant, more specifically a Brassica plant.

Said means thus include any means suitable for detecting the following Raphanus SNP markers within one or more of the following markers: SEQ ID NOs 4-22.

Any method known in the art may be used in the art to assess the presence or absence of a SNP. Some suitable methods include, but are not limited to, sequencing, hybridization assays, polymerase chain reaction (PCR), ligase chain reaction (LCR), and genotyping-by-sequence (GBS), or combinations thereof.

Different PCR based methods are available to the person skilled of the art. One can use the RT-PCR method or the Kaspar method from KBioscience (LGC Group, Teddington, Middlesex, UK).

The KASP^{™} genotyping system uses three target specific primers: two primers, each of them being specific of each allelic form of the SNP (Single Nucleotide Polymorpshism) and one other primer to achieve reverse amplification, which is shared by both allelic form. Each target specific primer also presents a tail sequence that corresponds with one of two FRET probes: one label with FAM^{®} dye and the other with HEX^{®} dye.

Successive PCR reactions are performed, the last one presence of the probes amplification. The nature of the emitted fluorescence is used to identify the allelic form or forms present in the mix from the studied DNA.

The primers identified in Table 3 are particularly suitable for use with the KASP^{™} genotyping system. Of course, the skilled person may use variant primers or nucleic acid probes of the primers as identified in Table 3, said variant primers or nucleic acid probes having at least 90%, and preferably 95% sequence identity with any one of the primers as identified in Table 3, or with the DNA genomic fragment amplified by the corresponding set of primers as identified in Table 3.

Percentage of sequence identity as used herein is determined by calculating the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. For example, nucleic acid sequences may be aligned using the BLAST 2 sequences (BI2seq) using BLASTN algorithms (www.ncbi.nlm.nih.gov).

As used herein, a primer encompasses any nucleic acid that is capable of priming the synthesis of a nascent nucleic acid in a template-dependent process, such as PCR. Typically, primers are oligonucleotides from 10 to 30 nucleotides, but longer sequences can be employed. Primers may be provided in double-stranded form though single-stranded form is preferred. Alternatively, nucleic acid probe can be used. Nucleic acid probe encompass any nucleic acid of at least 30 nucleotides and which can specifically hybridizes under standard stringent conditions with a defined nucleic acid. Standard stringent conditions as used herein refers to conditions for hybridization described for example in Sambrook et al 1989 which can comprise 1) immobilizing plant genomic DNA fragments or library DNA on a filter 2) prehybridizing the filter for 1 to 2 hours at 65°C in 6x SSC 5x Denhardt's reagent, 0.5% SDS and 20mg/ml denatured carrier DNA 3) adding the probe (labeled) 4) incubating for 16 to 24 hours 5) washing the filter once for 30min at 68°C in 6x SSC, 0.1% SDS 6) washing the filter three times (two times for 30min in 30ml and once for 10 min in 500ml) at 68°C in 2x SSC 0.1% SDS.

In specific embodiments, said primers for detecting the SNP markers of the present disclosure are as listed in the following table:

**Table 3: Primers for use in detecting Raphanus SNP markers of the invention (as indicated in the primer name)**

| SEQ ID | Nucleotide Sequence | Primer name |
|---|---|---|
| 64 | GAAGGTGACCAAGTTCATGCTCGAAGGGCGTAGTAGACGTTCA | SEQ ID NO :9_A1 |
| 65 | GAAGGTCGGAGTCAACGGATTGAAGGGCGTAGTAGACGTTCG | SEQ ID NO :9_A2 |
| 66 | CCTTGTTCCTCCTCACGCTCATAAT | SEQ ID NO :9_C |
| 67 | GAAGGTGACCAAGTTCATGCTCCACTGCACTTCCGGGTCATA | SEQ ID NO:8_A1 |
| 68 | GAAGGTCGGAGTCAACGGATTCCACTGCACTTCCGGGTCATC | SEQ ID NO :8_A2 |
| 69 | GAAGAATGCGACGAGGCTGTGCAA | SEQ ID NO :8_C |
| 70 | GAAGGTGACCAAGTTCATGCTAGAGAAAACGCAGAGGAGGCTTTA | SEQ ID NO :14_A1 |
| 71 | GAAGGTCGGAGTCAACGGATTGAGAAAACGCAGAGGAGGCTTTG | SEQ ID NO :14_A2 |
| 72 | GCGAACGGTTTGTTTTCCAATTACAGAA | SEQ ID NO :14_C |
| 73 | GAAGGTGACCAAGTTCATGCTCAAGTAGACCCAATAGTAGCGTCA | SEQ ID NO :19_A1 |
| 74 | GAAGGTCGGAGTCAACGGATTAAGTAGACCCAATAGTAGCGTCC | SEQ ID NO :19_A2 |
| 75 | ACCATCAACGCTGAGATCACACCAA | SEQ ID NO :19_C |
| 76 | GAAGGTGACCAAGTTCATGCTGGTACGGTTCCGGAGGTGGA | SEQ ID NO :21_A1 |
| 77 | GAAGGTCGGAGTCAACGGATTGTACGGTTCCGGAGGTGGC | SEQ ID NO :21_A2 |
| 78 | CGACCATTTCCGCTTCCATAACCAT | SEQ ID NO :21_C |
| 79 | GAAGGTGACCAAGTTCATGCTAGTACAAAGCTGAGTCAAGCA | SEQ ID NO :20_A1 |
| 80 | GAAGGTCGGAGTCAACGGATTCTAGTACAAAGCTGAGTCAAGCG | SEQ ID NO :20_A2 |
| 81 | CAGGAAGTGGATACTTGCGCTTGAT | SEQ ID NO :20_C |
| 82 | GAAGGTGACCAAGTTCATGCTTTATTGCCATTGCCATTATCCTTGGA | SEQ ID NO :17_A1 |
| 83 | GAAGGTCGGAGTCAACGGATTATTGCCATTGCCATTATCCTTGGG | SEQ ID NO :17_A2 |
| 84 | GTGACAGCAATGATCTTGACAAGATTGTA | SEQ ID NO :17_C |
| 85 | GAAGGTGACCAAGTTCATGCTAAGGTGTACTATTGCAAAGAAGACGAA | SEQ ID NO :12_A1 |
| 86 | GAAGGTCGGAGTCAACGGATTGGTGTACTATTGCAAAGAAGACGAG | SEQ ID NO :12_A2 |
| 87 | TCAAACTGAACAGACTGGTACAAGCAAA | SEQ ID NO :12_C |
| 88 | GAAGGTGACCAAGTTCATGCTGGGAGAGAAAGGCGCAGGTA | SEQ ID NO :6_A1 |
| 89 | GAAGGTCGGAGTCAACGGATTGGGAGAGAAAGGCGCAGGTT | SEQ ID NO :6_A2 |
| 90 | TTTTGTCAACCCCTGCTGTCGCTAA | SEQ ID NO :6_C |
| 91 | GAAGGTGACCAAGTTCATGCTCAAGATCTTGGGTGTGCCCACAA | SEQ ID NO :15_A1 |
| 92 | GAAGGTCGGAGTCAACGGATTCAAGATCTTGGGTGTGCCCACAT | SEQ ID NO :15_A2 |
| 93 | CTCCAGCAAGTGCATCTTCAATAACAATA | SEQ ID NO :15_C |
| 94 | GAAGGTGACCAAGTTCATGCTCGAAGATTCAATGATTCAAGTGACAC | SEQ ID NO :5_A1 |
| 95 | GAAGGTCGGAGTCAACGGATTCGAAGATTCAATGATTCAAGTGACAG | SEQ ID NO :5_A2 |
| 96 | GGTGTTGCAGCTCTTGAAGTTGGAA | SEQ ID NO :5_C |
| 97 | GAAGGTGACCAAGTTCATGCTGTAGCTGGAGTTCTTTTCATC | SEQ ID NO :16_A1 |
| 98 | GAAGGTCGGAGTCAACGGATTGGCTGTAGCTGGAGTTCTTTTCATT | SEQ ID NO :16_A2 |
| 99 | CCCTTGAGCTTATCTAAATCTTCGTTCTT | SEQ ID NO :16_C |
| 100 | GAAGGTGACCAAGTTCATGCTAGTAGCTCAAACCGCAATCATACCA | SEQ ID NO :13_A1 |
| 101 | GAAGGTCGGAGTCAACGGATTAGCTCAAACCGCAATCATACCG | SEQ ID NO :13_A2 |
| 102 | TTCCGAGACCGAGAATGTCTTGCAT | SEQ ID NO :13_C |
| 103 | GAAGGTGACCAAGTTCATGCTCAGTATTTGCTTACATATATCTCCAGTCA | SEQ ID NO :11_A1 |
| 104 | GAAGGTCGGAGTCAACGGATTGTATTTGCTTACATATATCTCCAGTCC | SEQ ID NO :11_A2 |
| 105 | CCGAAACGCTTTGGGAGACGATAAA | SEQ ID NO :11_C |
| 106 | GAAGGTGACCAAGTTCATGCTGGGTTCTTGAAGCAGCCTGAC | SEQ ID NO :10_A1 |
| 107 | GAAGGTCGGAGTCAACGGATTGGGGTTCTTGAAGCAGCCTGAT | SEQ ID NO :10_A2 |
| 108 | GGATCTCCTGCATCATGTTGACGAA | SEQ ID NO :10_C |
| 109 | GAAGGTGACCAAGTTCATGCTGAACGCACAGGTTTGCCACTGAA | SEQ ID NO :4_A1 |
| 110 | GAAGGTCGGAGTCAACGGATTAACGCACAGGTTTGCCACTGAG | SEQ ID NO :4_A2 |
| 111 | AGATGCAATTGTCACTCTTTCTCCTTCTT | SEQ ID NO :4_C |
| 112 | GAAGGTGACCAAGTTCATGCTCCATTGACACCAACGAACTCTTTAA | SEQ ID NO :7_A1 |
| 113 | GAAGGTCGGAGTCAACGGATTCCATTGACACCAACGAACTCTTTAC | SEQ ID NO :7_A2 |
| 114 | GTCTTGTTCTCAAGACCATCCCACTT | SEQ ID NO :7_C |
| 115 | GAAGGTGACCAAGTTCATGCTGCGCTAGTGACGTGAAGAAGAAA | SEQ ID NO :18_A1 |
| 116 | GAAGGTCGGAGTCAACGGATTGCGCTAGTGACGTGAAGAAGAAG | SEQ ID NO :18_A2 |
| 117 | GCTTCTTATACCCAAACCTCTCAATCAAA | SEQ ID NO :18_C |

### Use of Brassica plants of the disclosure

Brassica plants of the present disclosure may be used for breeding applications. As used herein, breeding applications encompass pedigree breeding to improve the agronomical value of a plant, line, hybrid, or variety. In specific embodiment, it relates to backcrossing activities in order to create new recombinant lines in a genomic region of interest or to introgress a region of interest in another plant not comprising such region. Typically, in the present disclosure, the Brassica plants are used to introgress the Raphanus region conferring POSH+ phenotype in another plant.

Accordingly, it is a further disclosed a method of production of a POSH+ Brassica plant, wherein the method comprises the following steps:
a. crossing a first Brassica plant as described in the previous section with a second POSH⁻ or POSH+ Brassica plant; thereby obtaining a F1 hybrid plant;
b. selfing or backcrossing said F1 hybrid plant with said second POSH⁻ or POSH⁺ Brassica plant;
c. selecting the POSH⁺ Brassica plant among the plant obtained in step b), optionally using at least one Raphanus SNP within at least one of the markers SEQIDN°19, SEQIDN°20 or SEQIDN°21 and
d. optionally, further selecting said POSH+ Brassica plant for the absence of at least one of the Raphanus SNPs within at least one of the marker SEQ ID NOs 4-18.

In a specific embodiment, a first plant as used in the above method is the plant obtained from a representative sample of the seeds as deposited on July 27, 2015 at NCIMB collection under the accession number 42444, obtained from Brassica napus R42141F as described in Example 5 below.

Any Brassica plants obtained or obtainable by the disclosed methods for producing Brassica plant with POSH+ phenotype are also part of the present invention.

Brassica plants disclosed herein are further useful for example for producing canola oils. Seeds harvested from plants described herein can be used to make a crude canola oil or a refined, bleached, deodorized (RBD) canola oil. Harvested canola seed can be crushed by techniques known in the art. The seed can be tempered by spraying the seed with water to raise the moisture to, for example about 8.5%. The tempered seed can be flaked using a smooth roller with, for example a gap setting of 0.23 to 0.27mm. Heat may be applied to the flakes to deactivate enzymes, facilitate further cell rupturing, coalesce the oil droplets, or agglomerate protein particles in order to ease the extraction process. Typically, oil is removed from the heated canola flakes by a screw press to press out a major fraction of the oil from the flakes. The resulting press cake contains some residual oil.

Crude oil produced from the pressing operation typically is passed through a settling tank with a slotted wire drainage top to remove the solids expressed out with the oil in the screw operation. The clarified oil can be passed through a plate and frame filter to remove the fine solid particles. Canola press cake produced from the screw pressing operation can be extracted with commercial n-Hexane. The canola oil recovered from the extraction process is combined with the clarified oil from the screw pressing operation, resulting in a blended crude oil.

The Brassica plants or their oil are also useful as food compositions, for human or animal. The oil may also be used in biofuel.

### EXAMPLES

### Example 1: Creation of new recombinant Brassica plant

Brassica napus is a relatively young crop and does still show some characteristics of wild species. One of these characteristics is a tendency for pod shattering at harvest time. It has been shown that some B. napus Ogura-hybrids show a much better pod shatter tolerance. In order to characterize this trait and obtain new recombinant lines, 204 crosses were done in January 2011. Here Ogura males and hybrids with the original Raphanus introgression and carrying the pod shatter tolerance have been crossed with Ogura males with shortened Raphanus introgressions or with inbred lines not carrying a Raphanus introgression. Moreover Ogura males with shortened Raphanus introgressions have been crossed with inbred lines not carrying a Raphanus introgression. In November 2011 the resulting F2 plants were genotyped using SNP markers located on C09 and flanking the Raphanus introgression. One SNP Marker is flanking the Raphanus introgression on telomeric region, four other SNP markers are flanking the Raphanus introgression on centromeric region.

Hence all marker profiles combinations of telomeric and centromeric SNP markers which were not present in the parents of the respective cross indicate a recombination between these markers and consequently probably within the Raphanus introgression located between these markers. By this approach 62 potential recombinant plants have been identified from 11770 F2 plants. Such screening was repeated in 2013, 2014 and 2015 selfed seed of all 62 potential recombinants from 2012 were sown in F3 to validate the results from F2 plants. F3 plants were analysed again with the telomeric and centromeric SNP markers flanking the introgression and SNP markers BnRfo5 (depicted as SEQIDN°1) and SSR markers C08 and Boljon (depicted as SEQ ID NO:2) located on the Raphanus introgression. F3 lines where the recombination was validated were continued to F4.

### Example 2 : New markers development

Characterization with molecular markers of the new recombinant plants is very difficult. Indeed, on one hand the introgression has replaced a part of the Brassica napus genome and it is difficult to find markers that work in both Raphanus and napus species. Moreover due to the low level of recombination rate in this region, it is not possible to map the position of markers on the introgression based on linkage. Therefore the possibilities to describe the introgression were very limited. To address the problem of SNP discovery, we employed a Next Generation Sequencing (NGS)-based approach on the transcriptome of vegetative tissue.

Specifically 118 fixed restorer lines and 27 fixed female lines were sampled at 4-weeks post emergence stage and flush frozen in preparation for RNA extraction. RNA concentration of each combined sample was measured using 1µl of each RNA sample on the Qubit fluorometer (Invitrogen). The current version Illumina mRNA-Seq kit was used according to the manufacturer's protocol to convert total RNA into a library of template molecules suitable for high throughput DNA sequencing for subsequent cluster generation. Libraries were prepared using 5µg of total RNA, with quantification and quality assessment being carried out by running 1µl of library on an Agilent DNA 1000 LabChip (Agilent Technology 2100 Bioanalyzer). The libraries were multiplexed two per lane, loaded onto the Illumina HiSeq2000 instrument following the manufacturer's instructions and run for 100 cycles (single end reads) to produce at least 2.0 Gb of sequence per sample.

In order to develop the markers of the discosure useful to identify new recombinant lines, initial sequence allignment and SNP discovery across the panel of lines was performed using MAQ (Li et al Genome Research 18:1851-1858, 2008) and Perl scripts (Trick et al Plant Biotech J. 7:334, 2009; Bancroft et al, 2011).

Across the 143 OSR lines for which sequence data were obtain, on average, 1.59 x 107 sequence reads of 100 bases (1.59 Gb sequence data) were aligned to 50.4 Mb of reference sequences, resulting in 31.5-fold coverage. As a result we developed a marker dataset comprising 84,022 simple SNPs (28,402 after removing those with minor allele frequency below 5%) and 119,523 hemi-SNPs (80,100 after removing those with minor allele frequency below 5%). The advantage of simple SNPs is that these markers can be assigned with more confidence to one of the two genomes of oilseed rape than can hemi-SNP markers. An analysis was conducted with the aim of identifying SNP markers associated with line designation of male parent (MP) or female parent (FP). 169 markers were identified that fully differentiated between the types. The 169 markers all shared the characteristic of the allele comprising an ambiguity code (i.e. indicating the presence of 2 bases) for MP lines and a resolved base for FP lines, consistent with the addition of an additional genomic segment (i.e. that associated with the CMS restorer locus from radish). The markers were clustered predominantly in two pairs of homoeologous regions on linkage groups A9 and C9, with a few in regions paralogous to these.

The inventors developed around 550 new SNP markers which are specific for the Raphanus introgression. By BLASTing these SNP to the oleracea genome it was concluded that the specific markers for the Raphanus introgression covers around 24 Mbp (length of the raphanus introgression) which is about 50% of chromosome C09.

Moreover we have identified a high number of markers which are functional in original B. napus but that are not present in Ogura-restorer lines. By BLASTing these to the oleracea genome it was found that these markers cover again around 24Mbp (lost B. napus chromosome segment). This was also validated by results from the Illumina 50k Chip array, where also Markers were not present in restorer material covering a fragment of about 22 Mbp.

This result clearly shows that one arm of the chromosome C09 was replaced by one arm of a Raphanus chromosome when the Ogura-introgression was created.

### Example 3: Phenotype characterization of the new recombinant lines

The stability of the pod can be measured with a test developed by Dr. Schulz at the Institute LFA-Mecklenburg-Vorpommern as described in March 2013 in Abschlussbericht 2013, Forschungsnummer 1/29, im Forschungskomplex:, Verfahrensoptimierung zur Verbesserung der Wirtschaftlichkeit (http://www.landwirtschaft-mv.de/cms2/LFA prod/LFA/content/de/Fachinformationen/Acker-und Pflanzenbau/Winterraps/Produktionstechnik/AB Platzfestigkeit 2013/Online For schungsbericht Platzfestigkeit 1 29.pdf). Pods are sampled at complete maturity (BBCH 97) from the middle part of the main stem. After sampling the pods are kept under dry conditions at room temperature for at least 21 days in order to ensure complete maturity of all pods. In the test the measured parameter for pod shatter tolerance is the tension measured to tear the two halves of the pod apart. For the measurement a Sauter Digital Force Gauge FK 50 was used. 20 individual pods of each genotype have been measured and the average of the 20 measurements was calculated.

Example of the results of these measurements made with different lines or hybrids are given in tables 4 and 5. The tables clearly show that the increased pod shattering tolerance is limited to some Ogura restorers and hybrids with a long introgression from Raphanus sativus and it can be concluded that the increased pod shattering tolerance is coded on the Raphanus introgression.

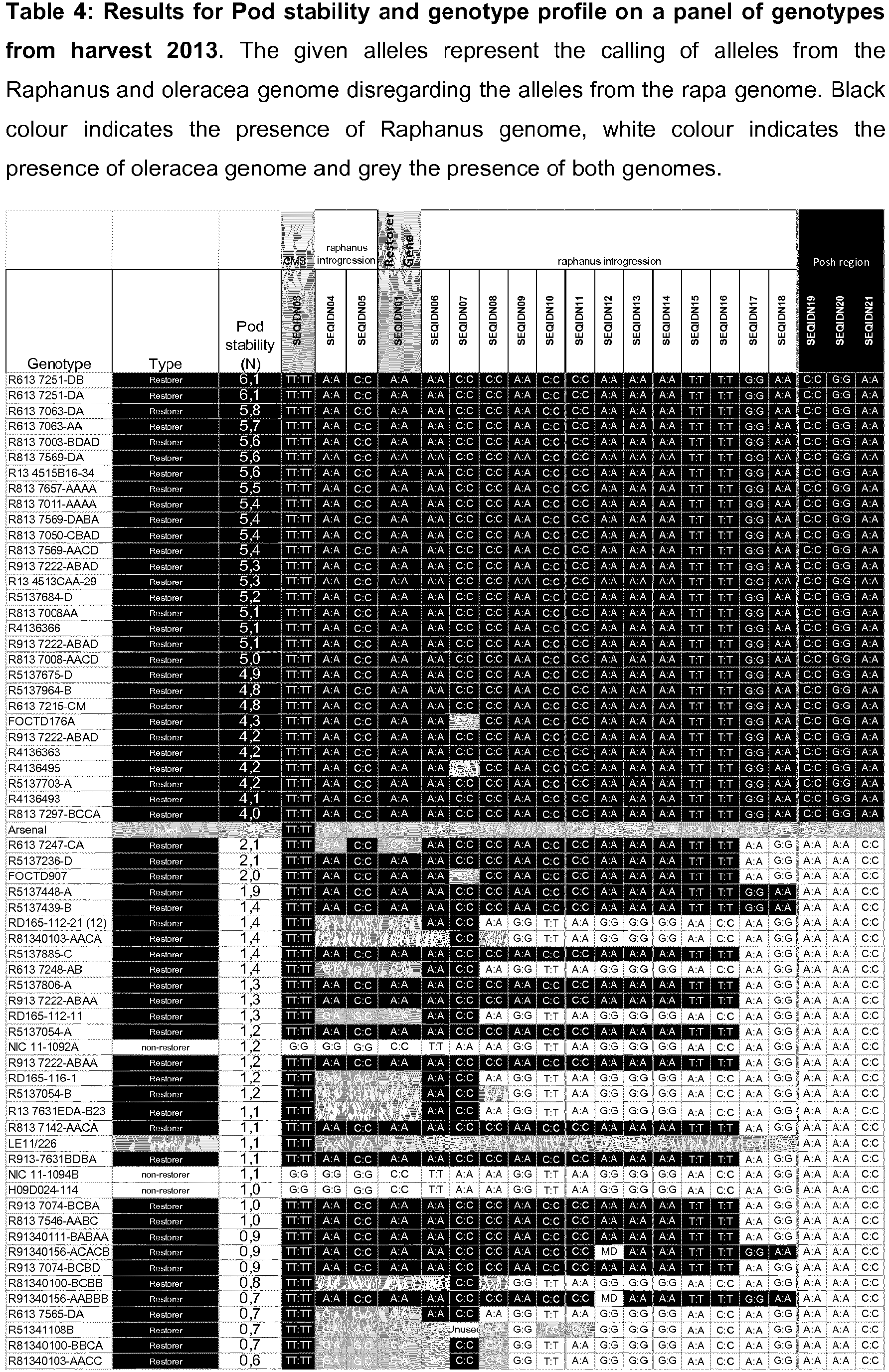

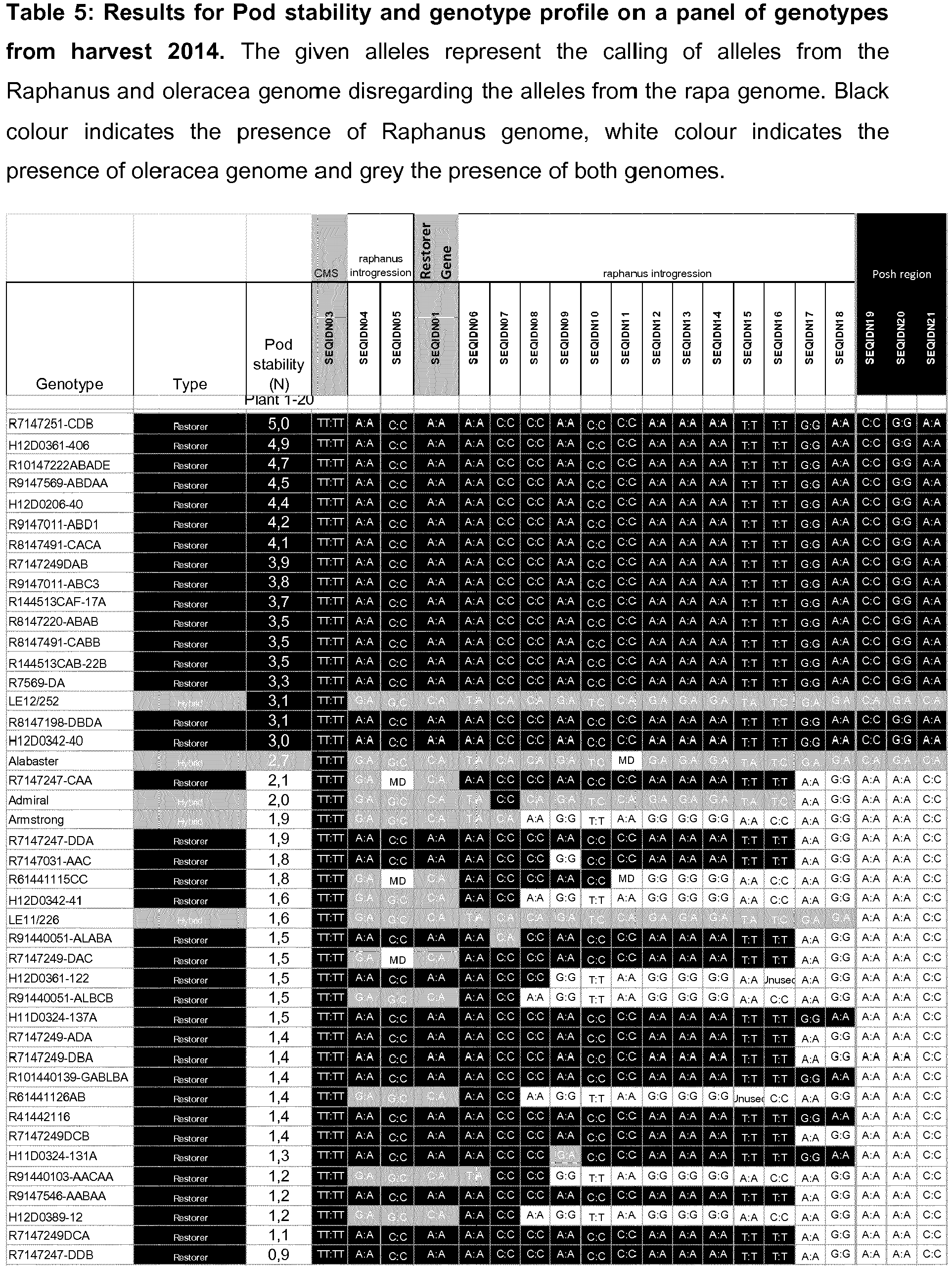

### Example 3: Pod shattering tolerance provided by Raphanus is partial dominant

Ogura restorers are used to produce hybrids with a sterile CMS line. In the resulting hybrid the Raphanus introgression is in the heterozygote state and therefore these hybrids are suitable to test if the pod shattering tolerance is inherited dominant, recessive or intermediate.

Examples of the results of the measurements of different Ogura hybrids are given in Figure 5. Black colour indicates the presence of Raphanus genome, white colour indicates the presence of oleracea genome and grey the presence of both genomes. For these measurements in total 100 pods were measured and the average of 100 measurements was calculated. The table clearly shows that the pod shattering tolerance is inherited at least partial dominant from long introgression Ogura restorers to the respective hybrids (genotypes 1-7 and 11). Genotypes 7 to 10 are also Ogura hybrids but for these the respective restorers are short introgressions lacking the pod shatter region from the Raphanus introgression. Consequently these hybrids do not show pod shattering tolerance.

### Example 4: Identification of other markers strongly associated to POSH locus:

The inventors have shown that surprisingly a FRUITFULL locus is localized on the Raphanus introgression as all the markers developed from the FRUITFULL gene sequence as identified on the Raphanus genome are strongly associated with the POSH locus markers described above (Figure 5).

In particular the inventors have also identified the predicted Open Reading Frame (SEQ ID NO:31) of the Raphanus FRUITFUL gene and the corresponding protein as predicted (SEQ ID NO:32) or corresponding predicted cDNA (SEQ ID NO:33). Such sequences may further advantageously be used to identify Raphanus SNP associated to POSH+ locus in Brassica plants.

Two different types of markers were identified. A first type is not genome specific. It is derived from a classic design with a SNP between napus and Raphanus, and a common marker shared with oleracea, rapa and radish. Thus, the one allele will amplify B.rapa and B.Oleracea, and the other allele is specific of the radish genome. In this type of design, the A genome is always amplified and therefore giving a background signal that decreases the resolution of the observations. This kind of marker does not permit us to distinguish AA/CC and AA/∅.

The second type of markers is genome specific. Therefore, there is no amplification of 'A' rapa genome. The design was realized between a SNP between napus and Raphanus and a HSV (Homeologous sequence variation) shared with oleracea and raphanus.

Examples of primers sequences to identify the non genome specific marker FRUITFULL_H1_04 are FRUITFULL_H1_04_F_A1 (SEQID N0 40), FRUITFULL_H1_04_F_A2 (SEQID N0 41) and (FRUITFULL_H1_04_F_C) SEQIDN0 42 and primers to identify the genome specific marker FRUITFULL_spe_01 are FRUITFULL_spe_01_R_A1 (SEQIDN0 52), FRUITFULL_spe_01_R_A2 (SEQIDN0 53) and FRUITFULL_spe_01_R_C (SEQIDN0 54).

These markers have been used to identify and follow the POSH region in breeding programs as shown in table 5.

### Example 5: Development of new pod shattering tolerant Brassica napus lines with shortened Raphanus introgression:

The F4 progeny of the lines obtained in example 1 was systematically phenotyped for pod shattering tolerance and screened with codominant SNP markers developed in example 3.

The following Table 6 show the SNP codominant markers which were used to analyze all the new recombinant plants generated:

| **SEQ ID NO** | **Nucleotide sequence** |
|---|---|
| **4** | TCAGACTCATCCAGATAAAGAAGAACAAAATCTCATCTTCTGTGCACTCTATGGTACAAAC TCCTTCAGGTACAGCWCGAACGCACAGGTTTGCCACTGA[A/G]ACAGCCGAGCTCCCTG CGCAAGAAGGAGAAAGAGTGACAATTGCATCTGCTGCTCCATCAGATGTTTACAGACAA GTGGGACCTTTCAAGTTTACCCCCA |
| **5** | TAAAGTATACTCGAAATGGCCCAAATCTCACTCTTTCAAGATCGGCGACTCCCTCTTGTTCT TGTACCCACCAAGCGAAGATTCAATGATTCAAGTGACA[C/G]CTTCCAACTTCAAGAGCT GCAACACCAAAGATCCGATCTTGTACATGAACGACGGCAACTCTCTCTTCAACCTCACCCA AAACGGAACCTTTTACTTCAC |
| **6** | GAAGTGTTCTGGACACAGCTGAGAAAGCCCACGAAGGGGATATCACATGCATTTCGTGG GCACCCAAGGCAATGACAGTTGGGGAGAGAAAGGCGCAGGT[A/T]TTAGCGACAGCAG GGGTTGACAARAAAGTGAAGCTGTGGGAAGCTCCAAMGTTGCAGTCTGTGTAGACTTGC |
| | TACTGCTGCTGCAATACAAAGAAAGTCT |
| **7** | CTAGTTTCAGGGAATGGTTTRCAGAAGGTTGAATTGATGAAGACGAGAGCTTCTTCATCA GACGAGACCTCAACGTCCATTGACACCAACGAACTCTTTA[C/A]WGACTTGAAGGAAAA GTGGGATGGTCTTGAGAACAARACRACYGTGGTTATCTAYGGAGGAGGAGCCATTGTWG CTGTTTGGTTATCTTCCATTCTTGTT |
| **8** | TCAAGAAGACTTACCCAACAGTCCAGCTTACAGCATGGACATTTTCCCCATTGTGGGATG GGTAAAYTACAAGTATGTGCCACTGCACTTCCGGGTCAT[C/A]TTGCACAGCCTCGTYGCA TTCTTCTGGGGAATCTTCCTGACCCTGCGAGCAAGGTCAATGACACTAGCTTTGGCAAAG GCTAAGTGATCAGGGAAACACA |
| **9** | AGTATGAAGAAGAGGGYGAGTATGAGAGAGGTGGGTCGAAGCAGAGGAGAGGAGAGT CAGAGGAAGGKCATGGRTACTACGAAGGGCGTAGTAGACGTTC[A/G]AGCCATTATGAG CGTGAGGAGGAACAAGGAGGTGASCAAGACCGKTACGAYGACCGTTATGGGAGAGTGG AGGAAGAAGAATACCGTTATGATGATCGTG |
| **10** | CTCCTCCKCCGAATCCGTTTGGGGAYGCGTTCAAGGGGCCMGAGATGTGGGCSAAGCTG ACGGCGGATCCGTCGACGAGGGGGTTCTTGAAGCAGCCTGA[C/T]TTCGTCAACATGAT GCAGGAGATCCAGAGGAACCCTAGCAGTCTCAATCTCTACTTGAAGGACCAGAGGGTGA TGCAGTCTCTYGGGGTTTTGTTGAATG |
| **11** | TCAAGGACTTTGGTGATAGTATTCCAGGACATGGTGGAATCACTGATAGAATGGACTGCC AGATGGTAATGGCAGTATTTGCTTACATATATCTCCAGTC[C/A]TTTATCGTCTCCCAAAG CGTTTCGGTTGACAAAATCCTGGACCAGATATTGACGAACCTTAGCTTCGAGGAACAACA AGCTCTCTTCACTAGATTAGGGC |
| **12** | TTGGCCCTGAAGGTTCTACAGTGCTTCATTATAGACAATCTTCAACTTCTGCTTCTATTGGG AAAATCAGTTGCAAGGTGTACTATTGCAAAGAAGACGA[A/G]GTTTGCTTGTACCAGTCT GTTCAGTTTGAGGTACCTTTCAAGRTGGAATCAGAAKCRTCTYCTTCYCAGGTGATCGCAT TCACCGTTAAACCTAGAGCAT |
| **13** | CTAAGGCAATGAAGTACCTGTCAATAGGTGAAGAAGACGATATATCATGGTCACTTATCA AAGCTGCCTTCTCTTCAGTAGCTCAAACCGCAATCATACC[A/G]ATGCAAGACATTCTCGG WCTYGGAAGTTCTGCCAGGATGAACACTCCAGCCACTGAGGTGGGGAACTGGGGTTGG AGGATTCCGAGTTCAACGAACTTTG |
| **14** | CTTTTGCTGGTTTTGGTGAAATAGTATCTGTCAAGATACCAGTTGGGAAAGGATGTGGAT TCATTCAGTTTGTCAACAGAGAAAACGCAGAGGAGGCTTT[A/G]GAGAAACTAAATGGT TCTGTAATTGGAAAACAAACCGTTCGCCTTTCMTGGGGTCGTAAYCAAGGCAAYAAACAG CCTCGAGGTGGGTATGG |
| **15** | GTCCATGTTTGATGCAATTGTATCAGCAGACGCATTTGAGAACTTGAAACCAGCTCCAGA TATTTTCTTGGCTGCTTCCAAKATCTTGGGTGTGCCCACA[T/A]GCGAGTGTATTGTTATT GAAGATGCACTTGCTGGAGTCCAGGCTGCTCAAGCTGCAAACATGAGATGCATAGCTGT GAAAACTACTTTATCTGAAGCAAT |
| **16** | ACTTTGTTGAYAGYCTTACMGGAGTAGGACTTGTTGATCAAATGGGAAACTTCTTCTGCA AAACGCTCTTGTTTGTGGCTGTAGCTGGAGTTCTTTTCAT[T/C]CGCAAGAACGAAGATTT AGATAAGCTCAAGGGTCTRWTYGAAGAGACGACGYTRTATGACAAGCARTGGCAAGCG GCTTGGAAAGAGCCGGAAATAATCA |
| **17** | CACAACATGCCGGTGATTGGTATCCAGCTGACCTTGGATCCAACGATTTCAAAGGTCTCTA TGGATATAAGGTCTTTATTGCCATTGCCATTATCCTTGG[G/A]GACGGTCTCTACAATCTT GTCAAGATCATTGCTGTCACTGTGAAGGAATTATGCAGCAATAGCTCTAGACACCTCAAT CTACCCGTTGTTRCCAACGTTG |
| **18** | AGAAGATGGAGTTCTTGATGTTTGATCTYGATCGGGTTTTGAARCCCGGTGGGTTGTTCT GGTTGGATAACTTCTACTGCGCTAGTGACGTGAAGAAGAA[A/G]GAGCTGACGCGTTTG ATYGAGAGGTTTGGGTATAAGAAGCTGAAATGGGTTATTGGAGAGAAGGCTGATGGGC AAGTGWATCTCTCTGCTGTTCTKCAAA |
| **19** | TAGAGCTGAAGCTAGGTATAGGAGGCACATCATAYAAAGATTTCATTCAAAGCCTTCATC TACCTATGCAATTGAGTCAAGTAGACCCAATAGTAGCGTC[C/A]TTCTCYGGAGGAGCTG TTGGTGTGATCTCRGCKYTGATGGTWGTWGAAGTCAACAACGTGAAGCAGCAAGAGCA CAAGAGATGCAAATACTGTCTAGGAA |
| **20** | TTAAGAACTGTGTCACTGACATTGACCCTGAGAGGGAGAAGGAGAAGAGAGAAAGGAT GGAAAGCCAAAACCTCAAGGCTAGTACAAAGCTGAGTCAAGC[G/A]AGGGAGAAAATC AAGCGCAAGTATCCACTTCCTGTTGCAAGGAGRCAACTYTCCACTGGRTACNTGGAAGAT GCTCTCGAAGAGGATGAAGAGACAGACC |
| **21** | GCTCAGGTAGATCTCCCACGGGTTGGGGAAGAGGATCCGGATATGGGTATGGGTCTGGA TCTGGATCAGGTAGCGGATATGGGTACGGTTCCGGAGGTGG[A/C]GGAGSACGTGGTG GTGGGTATGGTTATGGAAGCGGAAATGGTCGGTCTGGAGGWGGTGGTGGTGGCTCTAA TGGTGAAGTTGCCGCTTTGGGCCACGGTG |
| **22** | TCAGACTCATCCAGATAAAGAAGAACAAAATCTCATCTTCTGTGCACTCTATGGTACAAAC TCCTTCAGGTACAGCWCGAACGCACAGGTTTGCCACTGA[A/G]ACAGCCGAGCTCCCTG CGCAAGAAGGAGAAAGAGTGACAATTGCATCTGCTGCTCCATCAGATGTTTACAGACAA GTGGGACCTTTCAAGTTTACCCCCA |

The SNP is shown under bracket in the above marker sequences, the first nucleotide representing Raphanus SNP, the second nucleotide representing Oleracea SNP.

This systematic scoring resulted in the identification of one recombinant plant with a shortened raphanus introgression where the pod shatter coding region was still present. The F5 progeny of this plant is genotype R42141F with the pedigree (FOCTD909 x NSL09/196). This recombinant line is pod shattering tolerant and has a good pod size.

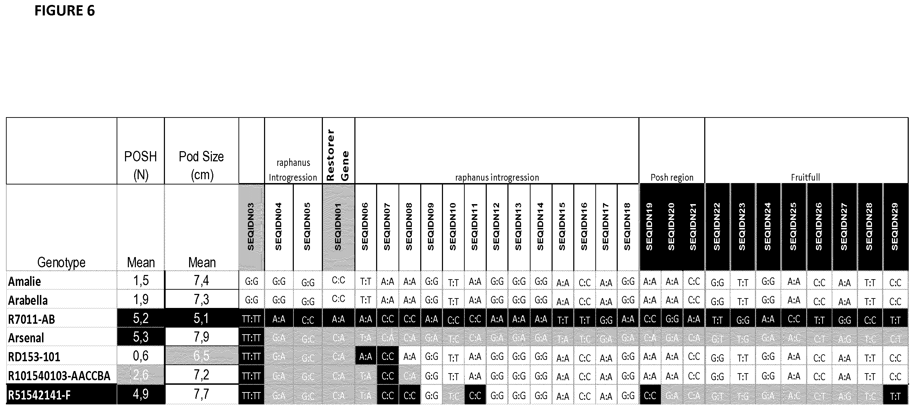

Figure 6 shows the results of the phenotyping and genotyping of the F5 progeny compared to other lines and hybrids. Amalie and Arabella are non restorer lines. R7011-AB is a restorer line with a long introgression comprising the Rf0 gene (BnRF0 marker as described in SEQIDN0:1) and the POSH region markers. Arsenal is a hybrid variety with a long introgression comprising the Rf0 gene (BnRF0 marker as described in SEQIDN0:1 ) and the POSH region markers. RD153-101 is a restorer line not pod shattering tolerant with a short introgression described in patent application WO2011020698. R101540103-AACCBA is a restorer line not pod shattering tolerant with a short introgression.

This result shows that the POSH region is localized in the region strongly associated with the POSH locus markers of SEQ ID NO:19, SEQ ID NO:20 and SEQ ID NO:21.

Results for Pod stability and genotype profile on the pod shattering tolerant recombinant line R51542141F (also called R42141F) and a panel of reference genotypes are shown in Figure 6. The given alleles represent the calling of alleles from the Raphanus and oleracea genome disregarding the alleles from the rapa genome. Black colour indicates the presence of Raphanus genome, white colour indicates the presence of oleracea genome and grey the presence of both genomes.

### Example 6: Identification of new pod shattering tolerant Brassica napus lines without Rf0 Raphanus region and obtention of pod shatter tolerant females and non Ogura inbred lines:

In order to create new recombinant restorer lines carrying shorter Raphanus introgression, 128 crosses have been done in January 2012. Here Ogura males and hybrids with the original Raphanus long introgression and carrying the pod shatter tolerance POSH⁺ have been crossed with POSH⁻ plants, Ogura males with shortened Raphanus introgressions or inbred lines not carrying a Raphanus genome fragment introgression. In November 2012, 6421 F2 plants resulting from these crossing were genotyped using 4 SNP markers located on C09 as described in "Exemple1". Selfed seed of all 353 potential recombinants were sown in F3 to validate the results from F2 plants. F3 plants were analysed in November 2013 with the set of codominant SNP markers developed in example 3.

The same SNP codominant markers were used to analyze all the new recombinant plants generated sowed in F3 in November 2013 (see previous Table 7).

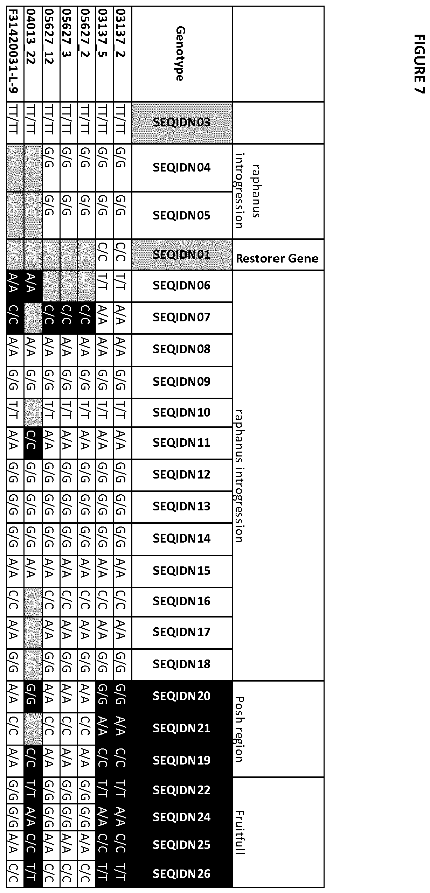

Among these F3 plants, the plants coded as FR-13C-3-03137-2 and FR-13C-3-03137-5 were identified. These plants were selected because they were carrying only the Raphanus favorable alleles of the markers SEQIDNO:19, SEQ ID NO:20 and SEQ ID NO:21 linked to pod shattering tolerance without any other raphanus alleles of the introgression. Since the plants were in sterile Ogura cytoplasm but did not contain the Rf0-gene, they were sterile and consequently could not be selfed. To maintain this event of recombination, 4 other recombinant fertile lines with shortened Raphanus introgression but carrying Rf0 gene were selected to be crossed with the sterile pod shatter tolerant recombinant (Genotypic profiles are given in Figure 7 which presents the molecular characterization of the F3 recombinant lines on the Raphanus introgression. Black colour indicates the presence of Raphanus genome, white colour indicates the presence of oleracea genome and grey the presence of both genomes.).

The resulting F1 is fertile, is carrying a shortened Raphanus fragment and the pod shatter tolerance. With this F1 it is possible on the one hand to develop inbred lines with shortened Raphanus introgression and carrying the pod shatter tolerance by inbreeding and marker assisted selection and on the other hand to cross the F1 as male parent and thereby transfer the pod shatter tolerance to any other Brassica plant. In this respect it is of special interest to cross the F1 to a Brassica napus plant to a fertile cytoplasm in order to transfer the pod shattering tolerance outside the sterile Ogura cytoplasm and to develop inbred lines with pod shattering tolerance but without the Rf0 gene. These inbred lines can subsequently be used as male/female parent in other hybrid systems (e.g. GMS) or after CMS-conversion as female parent in the Ogura hybrid system.

### Example 7: Correlation between the reduction of Raphanus introgression fragment and the increase of pod size

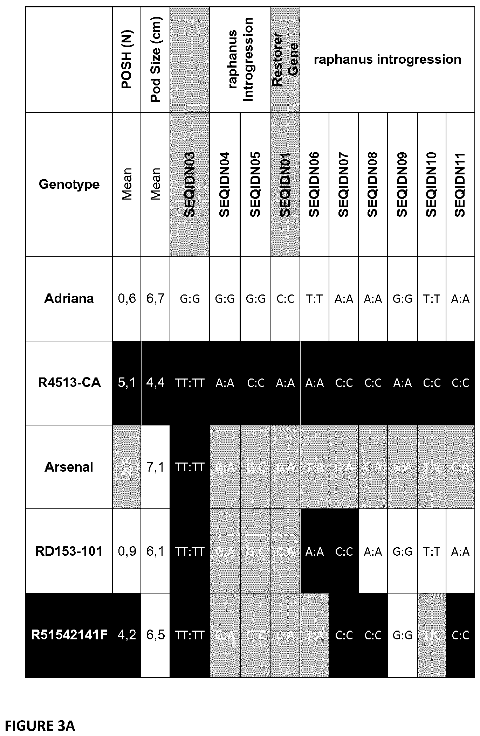

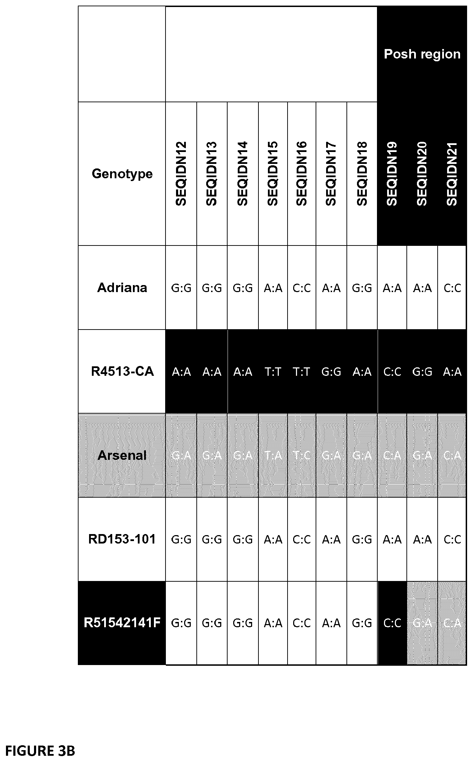

The pod size of different lines or hybrids carrying the POSH trait has been measured. Plants were grown in field and the pods were sampled at complete maturity. Pods size measurement corresponds to the measure (cm) of the upper half of the pod which does not comprise the beak and the pedicel. The results are shown in Figures 3A and 3B.

R51542141F is the recombinant line, R4513-CA is the line with the long introgression. Adriana is a control non restorer hybrid that does not comprise the Raphanus genome introgression.

Arsenal is a hybrid variety with a long introgression comprising the Rf0 gene and the POSH region markers. RD153-101 is a restorer line with short introgression which is not pod shattering tolerant.

The results show that there is a correlation between the reduction of Raphanus introgression fragment and the increase of pod size.

### Example 8 : segregation of POSH in a Double Hybrid population

Hybrids comprising the short introgression were crossed with hybrids comprising long introgression. Segregating DH-populations were generated and plants were grown in field. The pod stability was measured according to example 3.

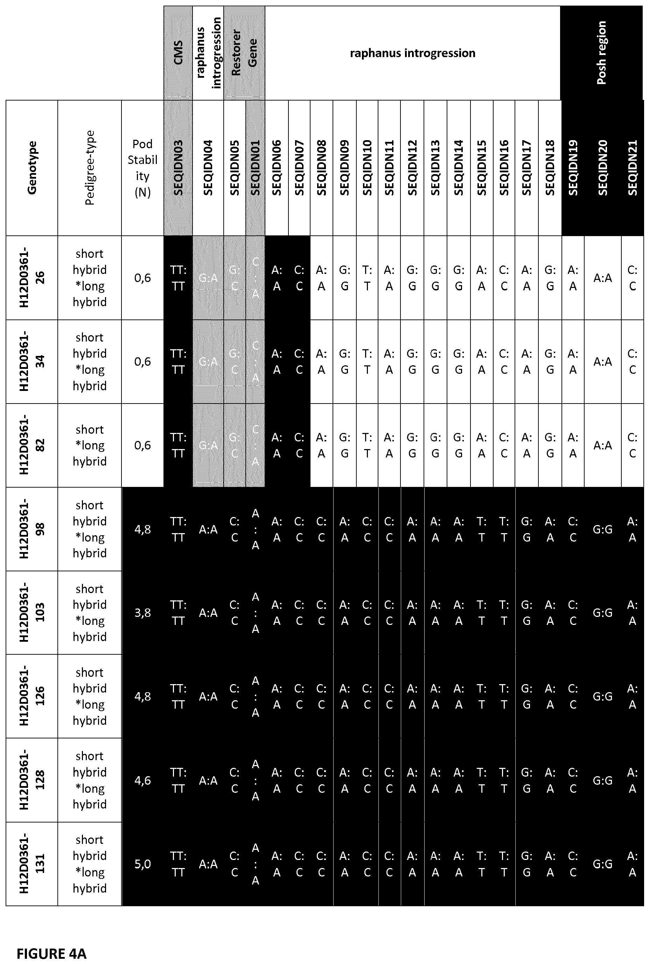

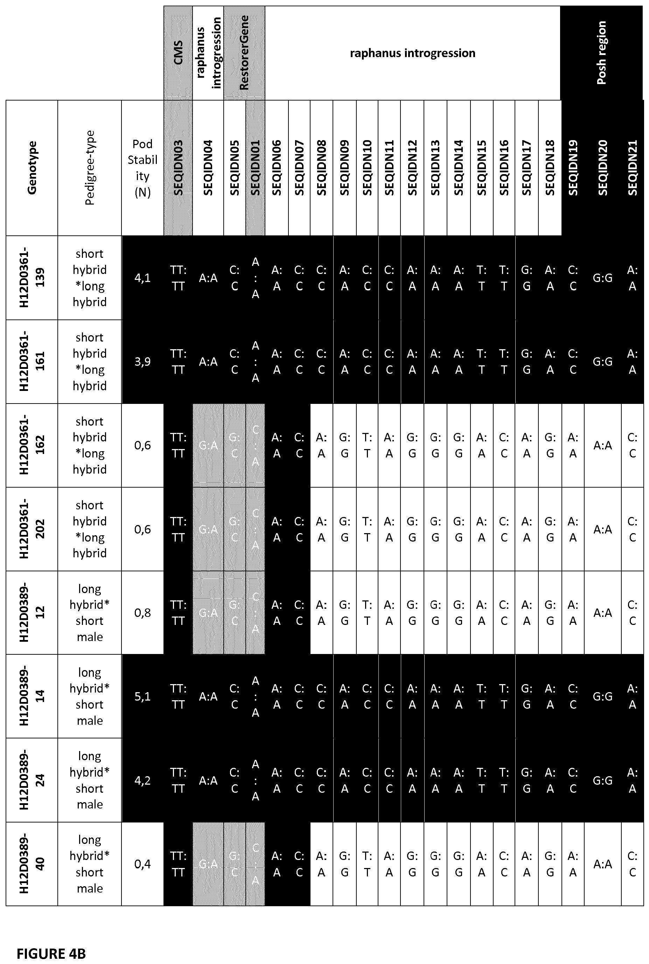

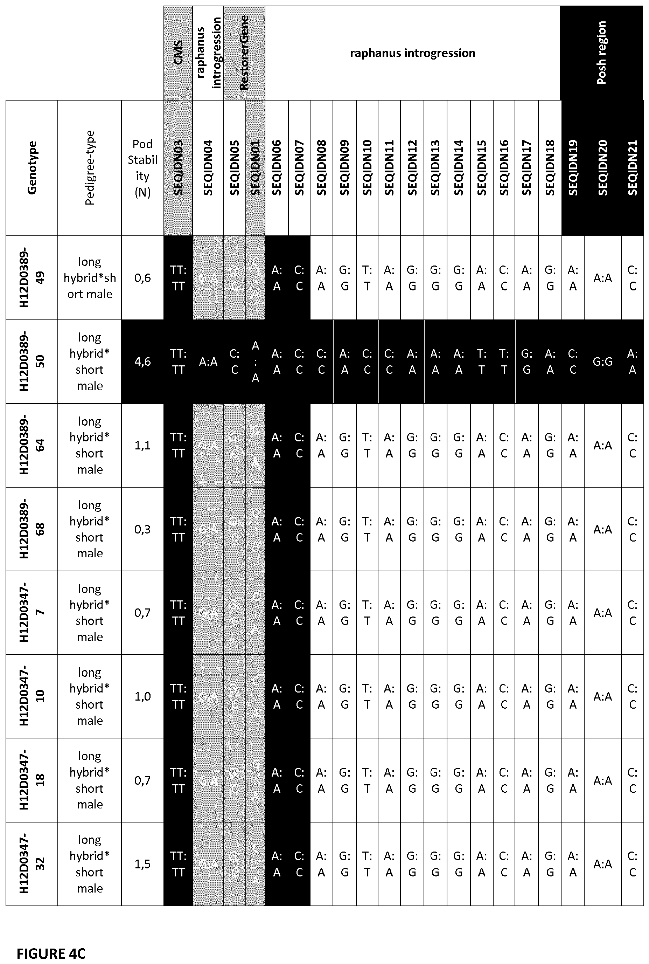

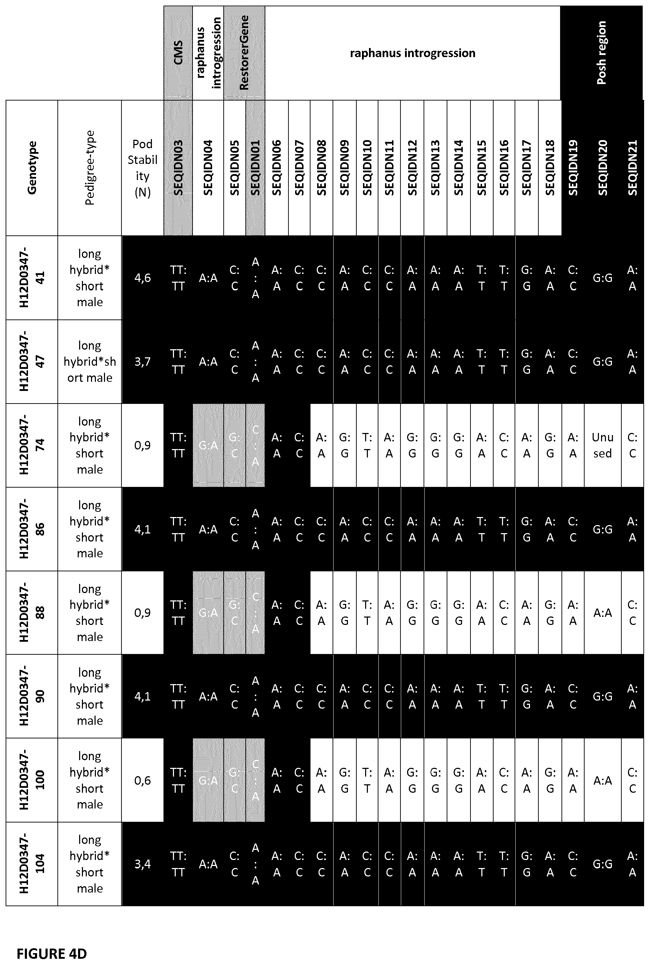

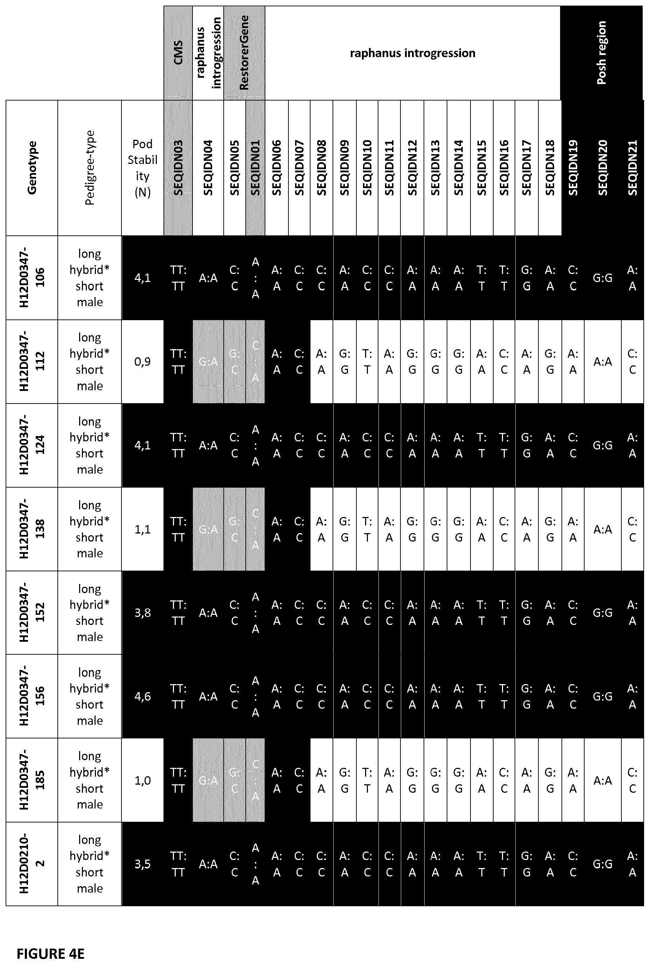

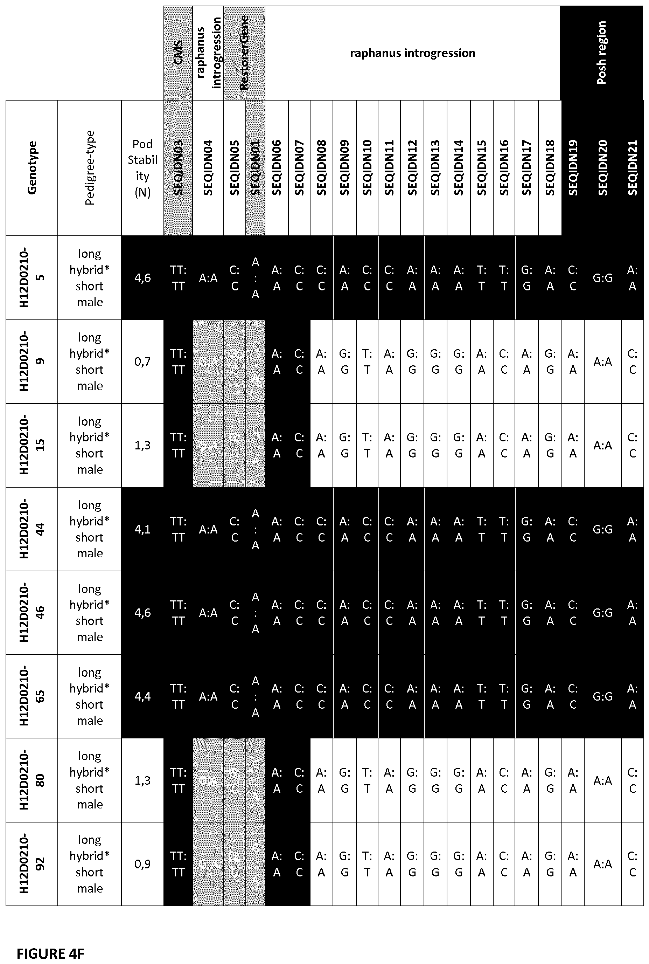

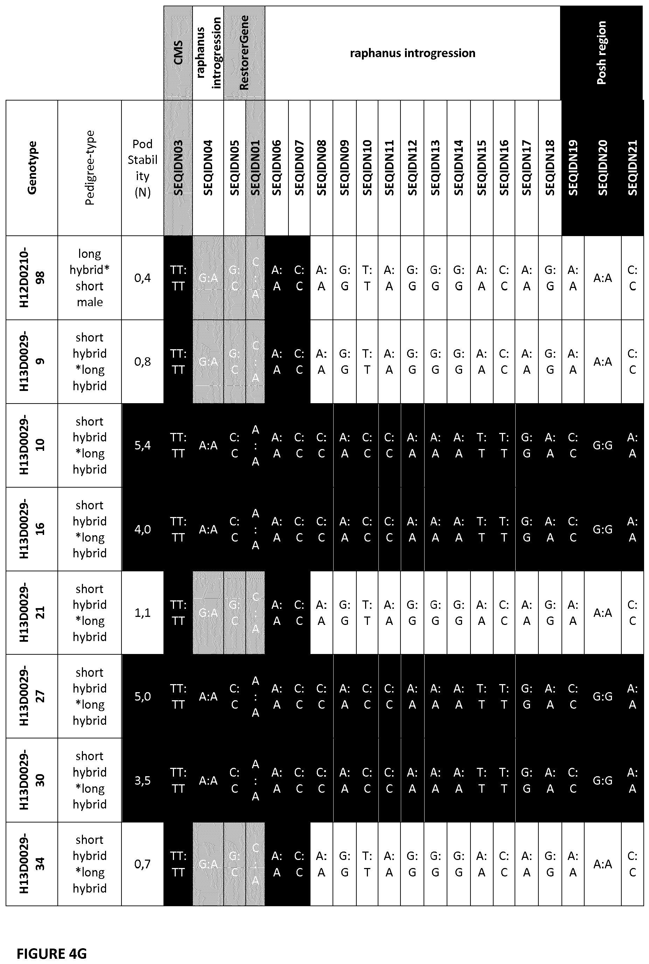

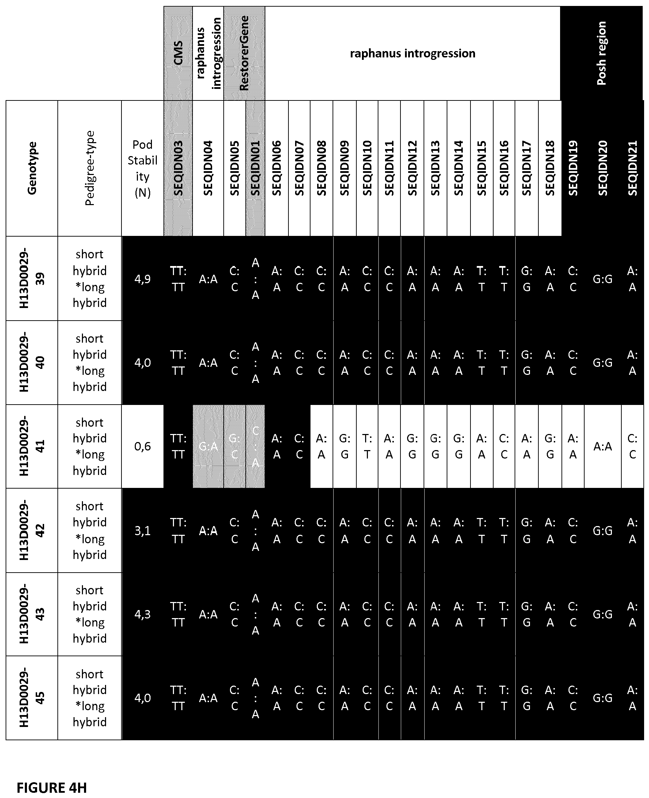

The results in Figures 4A to Figures 4H show the segregation of POSH in a Double Hybrid population.

## Claims

1. A Brassica plant comprising a Raphanus genomic fragment within its genome, wherein said fragment confers pod shattering tolerance phenotype POSH⁺ and said fragment is **characterized by** the absence of at least one Raphanus SNP within at least one of the following markers: SEQIDNOs 4-18.

2. The Brassica plant according to Claim 1, wherein at least one Raphanus SNP within at least one marker SEQIDN°19, SEQIDN°20 or SEQIDN°21 is present in said Raphanus genomic fragment.

3. The Brassica plant according to Claim 1 or 2, wherein the Raphanus SNPs within said markers SEQ ID NO:9 and SEQ ID NOs: 12-18 are absent.

4. The Brassica plant according to any one of Claims 1-3, wherein the Raphanus SNPs within said markers SEQ ID NOs:4-18 are absent.

5. The Brassica plant according to any one of Claims 1-4, wherein the plant further comprises a Raphanus FRUITFULL allele.

6. The Brassica plant according to claim 5, wherein the Raphanus FRUITFULL allele comprises the Raphanus SNP within marker of SEQ ID NO:22.

7. The Brassica plant according to any one of claims 1 to 6, wherein said plant further comprises the male fertility restoration locus Rf0 within the Raphanus fragment.

8. A Brassica plant according to any one of the claims 1 to 7 wherein said Brassica plant comprises a CMS Ogura cytoplasm.

9. A hybrid Brassica plant obtained by crossing a Brassica plant having a Raphanus fragment conferring POSH+ phenotype according to anyone of the claim 1 to 8, with another Brassica plant which does not have said Raphanus fragment conferring POSH+ phenotype, wherein said hybrid plant comprises the Raphanus genomic fragment which confers pod shattering tolerance phenotype POSH⁺.

10. A seed, a part of a plant, or a progeny of the Brassica plant according to any one of the claims 1 to 9.

11. Use of the Brassica plant or their seeds, according to any of the claims 1 to 10, for food applications, preferably for oil production and for feed applications, or for breeding applications, for example for use as a parent plant in breeding for improving agronomical value of a Brassica plant, line, hybrid or variety.
